# EUROPEAN PATENT SPECIFICATION

(11) **EP 1 818 416 B1**
(45) Date of publication and mention of the grant of the patent: **26.12.2012**
(21) Application number: 07103358.3
(22) Date of filing: 23.05.2005
(51) Int. Cl.: C12Q 1/68, C12Q 1/70

(54) **Methods and kits for the detection of HPV**
Verfahren und Kit zum Nachweis von HPV
Procédé et trousse pour la détection de papillomavirus humain (HPV)

(30) Priority: 21.05.2004 IT MO20040126
(43) Date of publication of application: 15.08.2007
(62) Divisional of application: 05011112.9
(73) Proprietor: BCS BIOTECH S.p.A., 09131 Cagliari (IT)
(72) Inventor: Perseu, Sinibaldo, 09045 Quartu Sant'Elena (CA) (IT); De Montis, Antonella, Cagliari (IT); Lautero, Cassandra, Cagliari (IT); Manca, Ilaria, Pirri (CA) (IT)
(74) Representative: Gervasi, Gemma

(56) References cited:
- WO-A-01/68915
- WO-A-01/73135
- WO-A-01/85994
- WO-A-03/014397
- WO-A-03/027323
- WO-A-03/057914
- WO-A-03/087829
- US-A- 4 889 818
- US-A- 5 565 320
- US-A1- 2002 132 227
- PATENT ABSTRACTS OF JAPAN vol. 2003, no. 12, 5 December 2003 (2003-12-05) & JP 2003 310257 A (YAMAGUCHI TECHNOLOGY LICENSING ORGANIZATION LTD), 5 November 2003 (2003-11-05)
- KLETER B ET AL: "Novel short-fragment PCR assay for highly sensitive broad-spectrum detection of anogenital human papillomavirus" AMERICAN JOURNAL OF PATHOLOGY, PHILADELPHIA, PA, US, vol. 153, no. 6, December 1998 (1998-12), pages 1731-1739, XP002100812 ISSN: 0002-9440
- KLETER BERNHARD ET AL: "Development and clinical evaluation of a highly sensitive PCR-reverse hybridization line probe assay for detection and identification of anogenital human papillomavirus" JOURNAL OF CLINICAL MICROBIOLOGY, WASHINGTON, DC, US, vol. 37, no. 8, August 1999 (1999-08), pages 2508-2517, XP002247920 ISSN: 0095-1137
- MOON ET AL: "521Development of oligonucleotide micro array for genotyping of sexually transmitted diseases" EUROPEAN UROLOGY SUPPLEMENTS, XX, XX, vol. 4, no. 3, March 2005 (2005-03), page 133, XP005006982 ISSN: 1569-9056
- DELRIO-LAFRENIERE S A ET AL: "LOW-DENSITY ADDRESSABLE ARRAY FOR THE DETECTION AND TYPING OF THE HUMAN PAPILLOMAVIRUS" DIAGNOSTIC MICROBIOLOGY AND INFECTIOUS DISEASES, ELSEVIER SCIENCE PUBLISHING CO., AMSTERDAM, NL, vol. 48, no. 1, January 2004 (2004-01), pages 23-31, XP001197394 ISSN: 0732-8893
- PARK TAE CHUL ET AL: "Human papillomavirus genotyping by the DNA chip in the cervical neoplasia." DNA AND CELL BIOLOGY, vol. 23, no. 2, February 2004 (2004-02), pages 119-125, XP002357729 ISSN: 1044-5498
- HWANG TAE SOOK ET AL: "Detection and typing of HPV genotypes in various cervical lesions by HPV oligonucleotide microarray." GYNECOLOGIC ONCOLOGY, vol. 90, no. 1, July 2003 (2003-07), pages 51-56, XP002357730 ISSN: 0090-8258
- KIM CHAN JOO ET AL: "HPV oligonucleotide microarray-based detection of HPV genotypes in cervical neoplastic lesions." GYNECOLOGIC ONCOLOGY, vol. 89, no. 2, May 2003 (2003-05), pages 210-217, XP002357731 ISSN: 0090-8258
- WILSON W J ET AL: "Sequence-specific identification of 18 pathogenic microorganisms using microarray technology" MOLECULAR AND CELLULAR PROBES, vol. 16, no. 2, April 2002 (2002-04), pages 119-127, XP001152529 ISSN: 0890-8508

## Description

The invention relates to a system for simultaneously searching for and identifying various pathogenic agents (bacteria, viruses or parasites) that are responsible for common and/or serious human pathologies, in particular pathogenic agents that are carried by water and/or foodstuffs such as meat, fish, milk, sweet foods or which may be found in biological samples of human origin such as pharyngeal swabs, urogenital swabs, bronchial aspirates, blood, biopsies and faeces.

The system can furthermore be used, by means of minimal modifications, for rapidly and simultaneously identifying polymorphisms and/or genetic alterations predisposing to certain neoplastic and/or genetic pathologies, e.g. familiar coagulation disturbances.

In the microbiological laboratories, identification of a pathogenic agent in a biological sample is usually performed in direct manner, that is through cultural examination of the sample.

A drawback of the aforementioned procedure consists of the fact that the latter, in addition to constituting a potential biological risk for the assigned operators, is often laborious, as in the case of the search for mycoplasms and enteric viruses, and requires at least 48-hour incubation of the sample. This increase in work time causes a consequent increase in analysis costs.

Furthermore, prolonged time taken to conduct the analysis leads to a significant delay in the delivery by a laboratory of the respective analytical result. This is potentially damaging, for example for a patient waiting to receive suitable treatment when the latter has to be based on the aforementioned analytical result.

Another drawback consists of the fact that many human and animal pathogenic agents are difficult or impossible to propagate in a culture, which makes it significantly difficult to apply the known analytical procedures.

A further drawback consists of the fact that often pathogenic agents that are able to produce similar clinical symptoms have very different culture features, which make it impossible to conduct a simultaneous search for these pathogenic agents by conducting a single analytical procedure.

In order to overcome the drawbacks of the prior art, microbiological analysis methods have been proposed that are based on molecular biology. The latter do not require the culture growth of the microorganism to obtain the identification thereof, as they are not based on the recognition of particular biochemical and/or serological features of the microorganism but on the identification of specific target gene sequences that are present in the genome of the microorganism. In order to obtain this identification, it is thus necessary to know, at least partially, the genome of the searched microorganism, and this is made possible by the fact that various databases exist from which the partial or complete sequences of most pathogenic agents are obtainable.

Identification methods have thus been devised that enable specific target sequences to be identified, if the latter are present in a sample, enable these sequences to be amplified by PCR technique and enable a final result (presence/absence) to be provided by analysing the amplified product by means of electrophoresis on agarose gel.

WO03027323 describes a DNA chip for detection of HPV infection and identification. of the HPV genotype, wherein the DNA chip comprises L1 specific probes. WO03057914 provides an *in vitro* method of screening for expression of mRNA transcripts from the L1 gene and the E6 gene of human papilloma virus in clinical samples.

Nevertheless, a drawback associated with the known methods based on PCR consists of the fact that the electrophoretic analysis alone does not prevent these methods, despite their considerable sensitivity, from providing misleading results,. i.e. false positives or false negatives.

A further drawback associated with the known methods based on PCR is that the amplification of genomic DNA is directed only to HPV specific L1 and E6 regions.

An object of the invention is to improve known microbiological analysis methods.

Another object is to provide an analytical method that enables pathogenic microorganisms that are taxonomically different from one another to be simultaneously searched for and identified in food and/or biological samples.

A further object is to provide an analytical method that enables microorganisms such as enteric viruses and mycoplasms to be searched for and identified in a substantially rapid manner, for which microorganisms significantly long and complicated methods are known and used.

Another further object is to improve analytical screening methods in the microbiological field.

A further other object is to provide a screening system for identifying pathogenic agents in food and/or biological samples.

A still further object is to provide a method for the interpretation of a result of a microbiological analysis.

In a first aspect of the invention, a method is provided for searching for and identifying pathogenic agents in a sample, comprising:
- extracting genomic nucleic acids from said sample;
- simultaneously amplifying a plurality of genic sequences comprised in said genomic nucleic acids, so as to produce amplified genic sequences;
- obtaining hybridization between said amplified genic sequences and specific probes bound to preset sites of miniaturized solid support means;
- highlighting said hybridization by means of a colorimetric reaction such as to give rise to a specific pattern on said miniaturized solid support means.

In an embodiment, the miniaturized solid support means comprises a chip.

In a second aspect of the invention, an analytical kit is provided for searching for and identifying pathogenic agents in a sample, comprising gene amplifying means for obtaining an amplification of specific genic sequences contained in nucleic acids of said pathogenic agents, miniaturized solid support means containing probes arranged to be hybridised with said genic sequences after said amplification, highlighting means arranged to highlight said hybridisation.

Owing to these aspects, a method, a chip and an analytical kit are made available that together constitute a screening system that enables various bacterial and/or viral pathogenic agents in a same sample to be searched for and to be rapidly identified. This screening system can be effectively used to analyse samples of potentially contaminated foodstuffs and biological samples of human origin and to search for those pathogenic agents, such as enteric viruses and mycoplasms, for which a search conducted through known analytical means involves elaborate procedures and a significant expenditure of time and money.

The highlighting means produces a colorimetric reaction that highlights the completed hybridisation of specific sequences with respective probes bound to the surface of the miniaturized solid support means, which can be the well of a glass poly-chip or the well of a microplate. This result can be read with the naked eye, if chips having an area of >1 cm² (poly-chip) are used or by means of a scanner if chips having an area of <1 cm² (microplate) are used.

The possibility of searching for and identifying a plurality of taxonomically distinct microorganisms in a same sample and during a single analysis arises from the fact that specific probes for: reciprocally different pathogenic agents can be fixed on the surface of a single chip, in numerous and preset positions (addresses) so as to form specific reciprocally different patterns. In this way, it is possible to simultaneously test for the presence of a vast range of specific sequences that are reciprocally different in a same amplified product and on a same chip.

Unlike what occurs in the methods that provide for the use of electrophoresis to analyse the PCR-amplified product, the method provided by the invention enables a substantially unequivocal result to be obtained and which is thus not interpretable in an incorrect manner. In fact, the amplified product obtained by PCR is hybridised with specific probes directed to "internal" sequences in relation to those used for amplification, which, in addition to increasing the sensitivity of the analytical method, enables the specificity of the latter to be significantly improved.

In a third aspect of the invention, a method is provided for interpreting an analytical result, comprising:
- carrying out a scan through optic reader means of a surface of a miniaturized solid support means, said surface comprising sites at which hybridisation between amplified genic sequences and specific probes has been induced, so as to obtain an image of said sites;
- comparing said image with a reference image, so as to verify whether and in which of said sites said hybridisation has been obtained.

In an embodiment, the interpreting method is performed by a programme that can be run on a computer system.

Owing to this aspect, it is possible to interpret precisely the results of an analysis conducted using chips having dimensions of <1 cm².

In another embodiment, the interpreting method provides for acquiring data that relate to the sample and are contained in barcode means using further optic reader means, and automatically associating such data with the analytical results obtained.

In this way, it is possible to manage jointly the data expressing the analytical results with other data relating, for example, to the type and/or the origin of an analysed sample, thus simplifying the management of the analytical results.

The invention will be better understood and implemented with reference to the attached drawings, which illustrate some exemplifying but non-limitative embodiments thereof, in which:
Figure 1 is a schematic exploded side view of a container for miniaturized solid supports;
Figure 2 is a schematic plan view showing an arrangement of specific probes on a miniaturized solid support;
Figures 3 to 32 are schematic plan views showing various patterns that are obtainable on the basis of the arrangement of specific probes in Figure 2;
Figure 33 is a schematic plan view showing a further arrangement of specific probes on a miniaturized solid support;
Figures 34 to 43 are schematic plan views showing different patterns that are obtainable on the basis of the further arrangement of specific probes in Figure 34;
Figure 44 is a schematic plan view showing a further other arrangement of specific probes on a miniaturized solid support;
Figures 45 to 50 are schematic plan views showing various patterns that are obtainable on the basis of the other further arrangement of the specific probes in Figure 36;
Figure 51 is an exploded perspective view of optic reader means;
Figure. 52 is another exploded perspective view of the optic reader means in Figure 51;
Figure 53 is a further exploded perspective view of the optic reader means in Figure 51;
Figure 54 is a diagram showing an arrangement of optic means comprised in the optic reader means in Figure 51;
Figure 55 is a schematic plan view of a microplate in which a plurality of miniaturized solid supports are obtained;
Figure 56 is a flow diagram showing the operation of the optic reader means in Figure 51;
Figure 57 is a flow diagram showing an operating arrangement comprising the optic reader means in Figure 51, further optic reader means and computer means;
Figure 58 is a flow diagram showing an operating phase of the operating arrangement in Figure 57;
Figure 59 is a flow diagram showing a further operating phase of the operating arrangement in Figure 57.

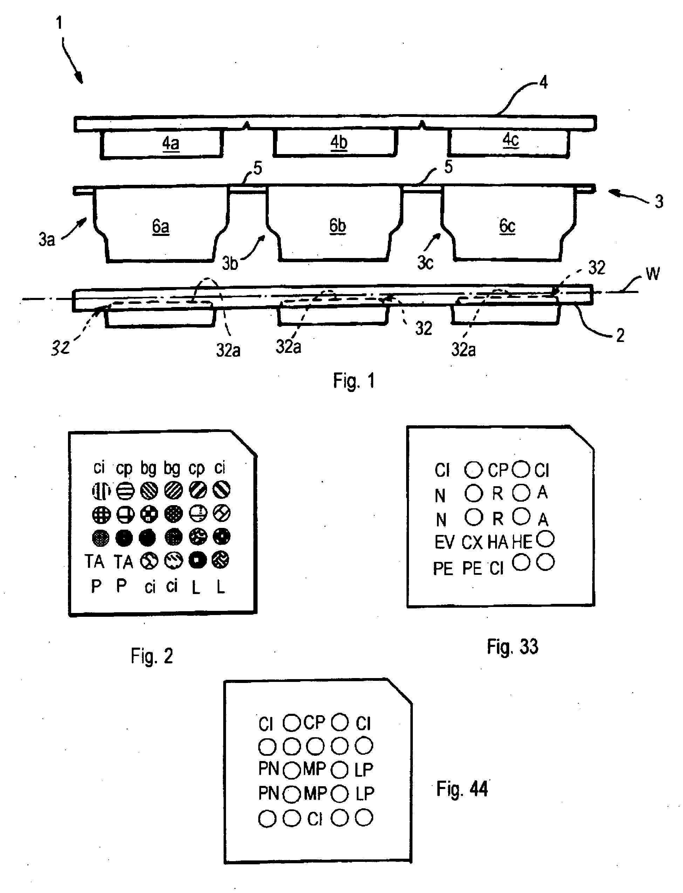

With reference to Figure 1, a container 1 is provided that is arranged to contain simultaneously three miniaturized solid supports, comprising a base 2, a seal 3 and a lid portion 4. In the rectangular base 2, three seats are obtained (that are not shown) that are reciprocally aligned according to and are arranged parallel to a longitudinal axis W of the base 2. In each seat a chip 32 is located (shown by a broken line) having an area of > 1 cm², arranged in such a way that a face 32a thereof faces in the direction opposite the base 2. The seal 3 comprises three hollow elements 3a-3c, each of which is approximately parallelepiped-shaped and is delimited by a respective side wall 6a-6c. The hollow elements 3a-3c are aligned parallel to the longitudinal axis of the base 2 and are connected together by bridge portions 5. The seal 3 can be fitted to the base 2 in such a way as to make each hollow element 3a-3c match a respective seat. In this way, each seat, and thus each chip 32 that is contained therein, is positioned on the bottom of a well defined by the side wall 6a-6c of the corresponding hollow element 3a-3c. The lid portion 4, which is rectangle-shaped, comprises three closing elements 4a-4b, aligned reciprocally and arranged parallel to the longitudinal axis W. Each closing element 4a-4b, which is quadrilateral-shaped, is positioned along the lid portion 4 in such a way as to be able to be inserted into a corresponding well, such as to close the latter when the lid portion 4 is applied to a side of the seal 3 opposite the base 2.

The container 1, in addition to the embodiment disclosed above comprising three chips 32 (poly-chips), can be made in an embodiment (not shown) that is arranged to house a single chip.

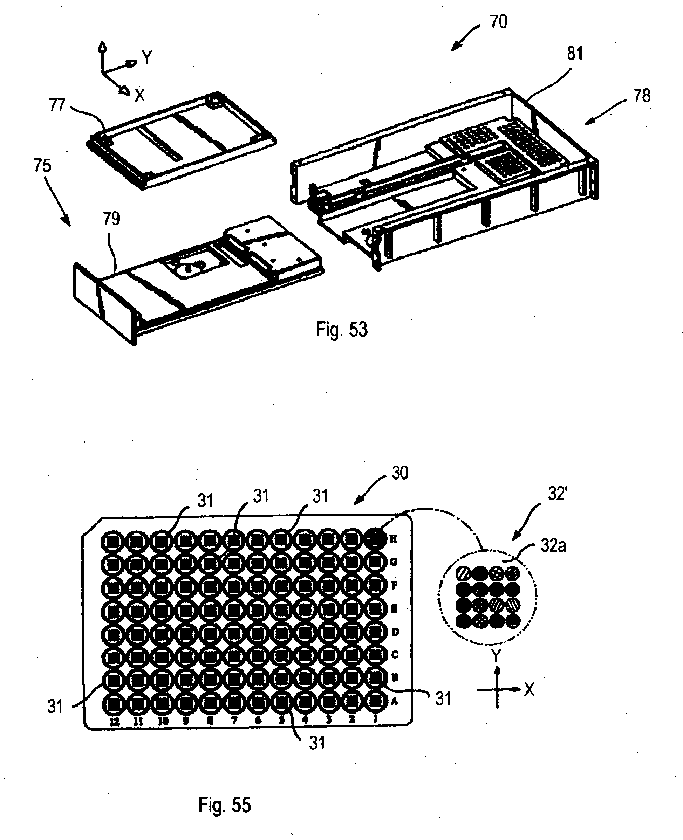

With reference to Figure 55, a microplate 30 is provided, having a rectangular plan shape, in which a plurality of wells 31 are obtained. On the bottom of each well 31 a chip 32' is obtained having an area of < 1 cm², the face 32a of which well faces a direction opposite the bottom of the well 31.

In an embodiment, the microplate 30 is obtained from a 96-well plate for microtitration (MTP) of the known type.

A variable and preset number of specific probes, comprised between a minimum of 2 and a maximum of 10000, is fixed to the face 32a of each chip 32, 32'. The probes may be spotted in preset positions or addresses of the solid support through one of the many commercially available apparatuses, for example the "SciFlex arrayer S11" (Scienion AG, Germany). This apparatus uses non-touch piezoelectric spotting technology and is able to spot a preset quantity of probes in each address in a reproducible and monitorable manner.

The bond of the probes on the solid support can be obtained both through covalent bonds and through high-affinity non-covalent bonds.

Covalent bonds can be obtained in different ways: by chemically modifying the oligonucleotides (for example, by adding amine, thiolic, mercapto or halogen groups), as disclosed by Guo et al. (1994) Nucleic Acid Res. 22: 5456-5465; by using substances with heterobifunctional reactive groups (such as 1-ethyl=3-(3-dimethylaminopropyl)-carbodiimide hydrochloride or EDAC), as disclosed by O'Donnel et al., (1991) Anal. Biochem 198: 138-142*;* Chrisey et al. (1996) Nucleic Acid Res.24: 3031-3039*;* Joos et al (1997) Anal. Biochem. 247: 96-101*;* with or without "post-spotting" treatment through 254 nm U.V. light or exposure to heat.

The halogen, amine, thiolic or mercapto group is added to the oligonucleotides in position 5'. Inserting a poly-T (polythymidine) tail that is between 5 and 20 mer in length in the oligonucleotides, still in position 5', is also provided for.

High-affinity non-covalent bonds can be obtained, for example, using biotinylated probes on supports modified by means of streptavidin.

The surfaces of the supports can, in turn, be chemically modified in different ways to promote the bond of the oligonucleotides (or of other macromolecules) by adding halogens, amine, or mercapto, functional groups as disclosed, for example, in: Lamture et al. (1994) Oligonucleotides Research 22: 2121-2125*;* Krieg et al. (1995) Nature 374: 546-549*;* Beattie et al. (1995) Mol. Biotechnol. 4: 213-225*;* Joos et al. (1997) Anal. Biochem 247: 96-101*;* Rogers et al. (1999) Anal. Biochem. 2 66: 23-30*;* Weiner et al. (1997) Proc. Natl. Acad. Sci. USA 94: 10833-10837*;* Boggs et al. (1997) Antisense Nucleic Acid Drug Dev. 7(5): 461-471*.*

The method provided by the invention above all requires extracting the genomic nucleic acids (DNA or RNA) from a sample to be analysed and/or from an enrichment broth, of the known type, in which the latter has been previously incubated. The sample may consist of water or of a foodstuff that can be contaminated, such as meat, fish, milk, sweet foods or may be a biological sample of human origin (pharyngeal swabs, bronchial aspirates, urogenital swabs, blood, biopsies, faeces).

Subsequently, simultaneous amplification of the various genic sequences present in the sample is provided for, which amplification is performed by means of multiplex PCR technique (which provides for the use of a set of primers that are specific for each of the target sequences searched for in the sample), if the genome of the pathogenic agent searched for is constituted by DNA or by means of multiplex RTPCR (Reverse Transcriptase PCR) technique, if the genome of the pathogenic agent searched for consists of RNA. The Multiplex PCR technique. The amplification procedure provides for the use of a pair of primers that amplify consensus regions for each of the target sequences searched for, that is for each pathogenic agent searched for. Each primer is an oligonucleotide that is able to trigger an amplification reaction catalysed by polymerase, at the end of which an amplified DNA is obtained. In order to be able to identify the amplified product obtained by PCR, marked primers are used, i.e. primers that have been modified in various ways. This can for example be obtained by inserting a biotin molecule, a digoxigenin molecule or a fluorochrome molecule on the end 5' of one or both the PCR primers.

Alternatively, the amplified product can be marked during PCR by incorporation of a nucleotide bound with a digoxigenin molecule or with another molecule that does not cause steric hindrance during the PCR reaction and subsequent hybridisation.

Once the amplified product has been obtained, the latter is denatured by heat treatment or by incubation in an alkaline environment. The denatured amplified product is then mixed with an appropriate hybridisation buffer and is essayed with the chip. Various hybridisation buffer (Maniatis) formulations exist, that basically vary from one another in their saline composition and in the type of "blocking" agent used to saturate the aspecific binding sites on the chip. Furthermore, "space-filling" macromolecules such as dextran sulphate are sometimes used to accelerate hybridisation kinetics.

Once the amplified product has been hybridised by the probe having a complementary sequence, this is highlighted by a technique that depends the manner in which the amplified product has been marked. If the latter has been marked with biotin or digoxigenin, the chip will be incubated with an antibody, conjugated for example with alkaline phosphatase or peroxidase, which antibody is directed against biotin or digoxigenin. Successive incubation of the chip with a specific substrate produces a colorimetric reaction in the exact site in which the enzyme and therefore the amplified hybridised product are present.

The analytical result can be read with the naked eye if chips 32 are used having an area of >1 cm². If on the other hand chips 32' are used having an area of <1 cm², an optic reader or scanner has to be used. The optic means vary according to the nature of the signal generated by the tracing molecule with which the amplified product has been marked. Tracing molecules that emit fluorescence require the use of sophisticated and expensive laser readers whereas the tracing molecules that develop a colorimetric reaction can be read by an optic reader using white light. In particular, an optic reader is provided (BCS Chip Reader) that enables the method for interpreting an analytical result provided by the invention and disclosed below to be applied.

Some examples are set out below in which the analytical system provided by the invention is applied to the search for bacteria and viruses in food, water and biological samples.

### Example 1

The method according to the invention has been used, in the form of an analytical kit, for searching and typing human Papillomavirus (HPV) in biological samples, comprising samples of exfoliated cervical cells and bioptic material fixed by formalin. Cancer of cervix uteri is still today the second cause of death due to tumours in the female sex. Epidemiological data clearly show that the occurrence of this tumour is closely linked to infections with some types of human Papillomavirus; the genome of this virus is found in nearly all cases of invasive cancer of the cervix. More than a hundred different types of HPV exist in nature, in addition to certain subtypes, and about thirty of these types are defined as anogenital because of their tendency to infect the mucose of the anogenital tract. Infection with genital HPVs is extremely frequent in the young sexually active population, but it fortunately clears up spontaneously and without leaving traces in the vast majority of cases. However, in a small percentage of cases, some genital HPVs, which are defined as "high oncogenic risk" HPVs, may establish persistent infections, causing neoplastic alterations to the mucous that may result in cancer. Nevertheless, such alterations may also persist for a long time without evolving and may even regress. The types of HPV considered to be high-risk are mainly represented by types 16, 18 and 45, which are the types most frequently found in invasive carcinomas. Other HPVs considered to be high-risk but which are less frequent than the former are types 26, 30, 31, 33, 35, 39, 51, 52, 53, 56, 58, 59, 66, 67, 68, 69, 70, 73 and 82. Genital HPVs are also known that never or only rarely cause invasive cancer. These are the low-risk HPVs, among which the types 6, 11, 34, 40, 42, 43, 44, and 54 are included. Some of these low-risk HPVs cause benign neoplasias such as warts and condylomas.

Cervical cancer evolves from dysplastic preinvasive lesions known as SIL (squamous intraepithelial lesions) or CIN (cervical intraepithelial lesions), which are classified in increasing degrees according to the seriousness of the alterations that are observed by microscopic analysis thereof. These lesions are usually discovered and possibly removed by surgery during normal gynaecological examinations. Nevertheless, since only a small number of these lesions is able to evolve, as already said, the aforementioned surgery is superfluous in most cases. The PAP test and colposcopy are essential for the diagnosis of these lesions, but unfortunately they cannot provide any type of information on their evolutive power.

However, it is known that the evolutive destiny of a given lesion depends on the type of HPV involved in the infection and on the duration of the infection. HPV typing is normally carried out by direct sequencing through hybridisation with type-specific molecular probes directed against the L1 viral region. A limit to this approach is constituted by the fact that the L1 region may be deleted during the process of integration of the viral DNA into the genome of the host cell, an essential stage in the viral transformation mechanism. If deletion of the L1 region occurs, the search for HPV and the corresponding typing provide an erroneously negative result, although the oncogenic power of the virus remains intact as it depends on the integrity of the E6 and E7 regions.

By applying the method provided by the invention, it is possible to significantly improve laboratory diagnostic procedures inasmuch as the HPV search and typing do not depend exclusively on the amplification of the L1 region. This result is obtained by using multiplex PCR, which enables the L1 and E6/E7 regions of high- and low-risk HPVs to be simultaneously amplified, followed by hybridisation with specific probes contained, for example, in the chips 32' of the microplate 30. Many primers have been tested to amplify the L1 region of the HPV. The set of GPF/GP6+ primers was chosen because it has a wide spectrum, i.e. it is able to identify a wide range of HPVs, producing an amplified product of approximately 190 pairs of bases. This constitutes an excellent compromise between the need to increase the efficiency of the PCR (which is inversely proportionate to the length of the product) and the need to have a sufficiently long product for unequivocal typing of the latter. The sequences of the primers used for the amplification of the E6/E7 region of the low- and high-risk HPVs are disclosed in MO2000A000091 and in W00185994.

In the reaction mixture there are also present the b-GLOBL2 and b-GLOBR2 primers that are specific for human β-globin. The presence of these primers enables the quality of the nucleic acids preparation to be monitored, which preparation undergoes PCR. In fact, in the presence of an unsuitable preparation, no signal will be obtained for the β-globin. Furthermore, the presence of inhibitors of the PCR reaction is monitored owing to the presence of an Internal Control (IC) that is amplified by the β-globin primers. The two PCR products are nevertheless distinguishable through hybridisation, as the internal control possesses nucleotidic sequences that are not present in the β-globin. All the primers have been synthesised in such a way as to be provided with a tail of 11 Thymidine residues at the end 5', with which a digoxigenin molecule is bound. Tables 1-3 below respectively show the sequences of the primers and of the probes for searching for and typing HPV and the sequences amplified by the b-GLOBL2 and b-GLOBR2 primers.

**Table 1 (primers)**

| | |
|---|---|
| GPF | digoxigenin-5'-CAG-GGA-CAI-AAI-AAT-GGY-ATW-TG-3' |
| GP6+ | digoxigenin -5'-GAA-AAA-TAA-ACT-GTA-AAT-CAT-ATT-3' |
| pu-2R | digoxigenin -5'-GAG-CTG-TCG-CTT-AAT-TGC-TC 3' |
| ELRF | digoxigenin -5'IWA-CIG-TGG-AAG-AAG-ARA-C-3' |
| EHRF | digoxigenin -5'-AGA-YRT-TAT-AIT-TAT-TCI-GTR-TAT-GG -3' |
| b-GLOBL2 | digoxigenin -5'-CTT-TCA-GGG-CAA-TAA-TGA-3' |
| b-GLOBR2 | digoxigenin -5' -TGG-TAG-CTG-GAT-TGT-AGC-3' |

The GP primers identify a zone of about 190 pb on HPV region L1. The probes indicated below are comprised within this region. The pu-1M, -31B, 2R primers amplify consensus regions in the E6/E7 region of high-risk HPV types (pu2R-pu31B) and in the E6/E7 region of low-risk HPV types (pu2R-pu1M), forming amplified products with a molecular weight between 270-300 pb. The b-GLOBL2 and b-GLOBR2 primers amplify a segment of 178 pairs of bases of the gene of the β-globin (access number NG000007).

**Table 2 (probes)**

| Target | Specificity | Oligonucleotide 5' → 3' sequence | mer |
|---|---|---|---|
| 1. | HPV6 | ATCCGTAACTACATCTTCCACATACACCAA | 30 |
| 2. | HPV11 | ATCTGTGTCTAAATCTGCTACATACACTAA | 30 |
| 3. | HPV16 | GTCATTATGTGCTGCCATATCTACTTCAGA | 30 |
| 4. | HPV18 | TGCTTCTACACAGTCTCCTGTACCTGGGCA | 30 |
| 5. | HPV26 | TAGTACATTATCTGCAGCATCTGCATCC | 28 |
| 6. | HPV30 | TATCTGCAACCACACAAACGTTATCCAC | 28 |
| 7. | HPV31 | TGTTTGTGCTGCAATTGCAAACAGTGATAC | 30 |
| 8. | HPV33 | TTTATGCACACAAGTAACTAGTGACAGTAC | 30 |
| 9. | HPV34 | TACACAATCCACAAGTACAAATGCACCATA | 30 |
| 10. | HPV35 | GTCTGTGTGTTCTGCTGTGTCTTCTAGTGA | 30 |
| 11. | HPV39 | TCTACCTCTATAGAGTCTTCCATACCTTCT | 30 |
| 12. | HPV40 | GCTGCCACACAGTCCCCCACACCAACCCCA | 30 |
| 13. | HPV42 | CTGCAACATCTGGTGATACATATACAGCTG | 30 |
| 14. | HPV43 | TCTACTGACCCTACTGTGCCCAGTACATAT | 30 |
| 15. | HPV44 | GCCACTACACAGTCCCCTCCGTCTACATAT | 30 |
| 16. | HPV45 | ACACAAAATCCTGTGCCAAGTACATATGAC | 30 |
| 17. | HPV51 | AGCACTGCCACTGCTGCGGTTTCCCCAACA | 30 |
| 18. | HPV52 | TGCTGAGGTTAAAAAGGAAAGCACATATAA | 30 |
| 19. | HPV53 | ACTCTTTCCGCAACCACACAGTCTATGTCT | 30 |
| 20. | HPV54 | TACAGCATCCACGCAGGATAGCTTTAATAA | 30 |
| 21. | HPV56 | GTACTGCTACAGAACAGTTAAGTAAATATG | 30 |
| 22. | HPV58 | ATTATGCACTGAAGTAACTAAGGAAGGTAC | 30 |
| 23. | HPV59 | TCTGTGTGTGCTTCTACTACTTCTTCTAT | 29 |
| 24. | HPV66 | ACTATTAATGCAGCTAAAAGCACATTAACT | 30 |
| 25. | HPV67 | ACTTTATGTTCTGAGGAAAAATCAGAGGCT | 30 |
| 26. | HPV68 | TTTGTCTACTACTACTGAATCAGCTGT | 27 |
| 27. | HPV69 | AGTACTGTATCTGCACAATCTGCATCTGCC | 30 |
| 28. | HPV70 | ATTGTCTGCCTGCACCGAAACGGCCAT | 27 |
| 29. | HPV73 | AGGCTAGTAGCTCTACTACAACGTATGC | 28 |
| 30. | HPV82 | GTTTACTCCATCTGTTGCACAAACATTTAC | 30 |
| 31. | β-globin | CAATGTATCATGCCTCTTTGCACCATTCTA | 30 |
| 32. | Internal control | GGGTTACGTAAGCTACCTAGCTACTGCATG | 30 |
| 33. | PAN HPV L1 | TTTGTWACTGTIGTRGAYACIACMCGIAGTAC | 33 |
| 34. | HPVHR E6/E7 | CGTACAGAAACCCAGGTGTAATCATGCRTG | 30 |
| | | CAGTATAATCATGCAYGGTAAAGTACCAAC | 30 |
| | | GTGTAATAAMGCCATGCGTGGTAATGTACCACA | 33 |
| | | ACGIAGIGAAACACAIGTITATTTTTTTATGCATGGACC | 39 |
| 35. | PAN HPV E6/E7 | TGYCAIAARCCITTRTGIMIAGIRGAAAA | 29 |

**Table 3 (amplified sequences of β-globin gene)**

| Target | Name of the Primer | Sequence 5'-3' |
|---|---|---|
| B-globin Reference sequence NCBI NG000007 | b-GLOBL2 | |
| | b-GLOBR2 | |
| Internal Control | b-GLOBL2 | |
| | b-GLOBR2 | |

The composition and the analytical procedure provided for a kit (BCS HPV KIT) intended for HPV search and typing are disclosed below.

### Composition of the kit

The kit comprises a Part A and a Part B. Part A comprises the reagents for the genic amplification, which reagents have to be stored at a temperature of < -20°C. Part B comprises the reagents for hybridisation and colorimetric detection on chip, which have to be stored at 2-8°C. In tables 4 and 5, set out below, the compositions of respectively Part A and Part B (for a 24-test kit) are shown in detail.

**Table 4 (Part A)**

| **N°** | **Solution** | **Solution** | **Volume** | **Quantity** |
|---|---|---|---|---|
| 1 | **FM** | PCR premix for amplifying the L1 and E6/E7 regions of a wide range of HPV types, an endogenous internal control DNA and a synthetic internal control DNA; it consists of primers modified in 5' by digoxigenin, deoxyribonucleotides (dNTPs), PCR buffer, MgCl₂ and water. | | |
| | Consisting of: | | | |
| | -DNTP 2.5 mM 1% | | | |
| | -10xPCR Buffer 12.67% | | | |
| | -MgCl2 (25mM) 12.67% | | | |
| | -Oligonucleotides 4% : | | | |
| | primer GPF (100 µM) | | | |
| | primer GP6+ (100 µM) | | | |
| | primer pu-2R (100 µM) | | | |
| | primer ELRF (100 µM) | | | |
| | primer EHRF (100 µM) | | 1.4 mL | 1 vial |
| | primer b-GLOBL2 (100 µM) | | | |
| | primer b-GLOBR2 (100 µM) | | | |
| 2 | **DNA Polymerase** 100% | DNA Polymerase | | |
| | | Enzyme(5U/µL) | 24 µL | 1 vial |
| 3 | **IC**, consisting of DNA IC 7% in water | PCR internal control | 100 µL | 1 vial |
| 4 | **CP**, consisting of HPV 42 DNA 7% in water | PCR positive control | 100 µL | 1 vial |

**Table 5 (part B)**

| N° | Solution | Description | **Volume** | **Quantity** |
|---|---|---|---|---|
| 1 | BCS Hyb Buffer Consisting of: | Hybridizating buffer that is formulated so as to promote the hybridization of the amplified products with the specific probes | 20 mL | 1 bottle |
| | Maleic acid 1x 11.73 % | | | |
| | NaCl 2.5 M 27.72% | | | |
| | Boric acid 500 mM 1.56% | | | |
| | NaOH IN 6.27% | | | |
| | Dextr. Sulf. Sodium 50.58% | | | |
| | Herring Sperm DNA(10 g/bt) | | | |
| | Ficoll TM PM400 1.01% | | | |
| | PVP 1.01% | | | |
| | Anti-VP1(100 µM) 0.1% | | | |
| | Wash Buffer Consisting of: | Washing buffer for removing the amplified products that are not bound after the hybridization phase | 80 mL | 1 bottle |
| | NaH2PO₄.2H₂O 1M 0.15% | | | |
| | Na₂HPO₄.2H₂O 1M 0.54% | | | |
| | NaCl 2.5 M 0.78% | | | |
| | MgCl₂. 1M 0.18% | | | |
| | Urea 1% 7.69% | | | |
| 3 | Anti-Digoxigenin Antibody conjugated with Alkaline phosphatase (Roche Molecular Diagnostics) | The antibody binds specifically the digoxigenin of the amplified products hybridizated with the probes. The conjugated alkaline phosphatase (AP) is the enzyme that, in the presence of NBT/BCIP substrate, enables a colorimetric signal to be developed on the chip. | 24 µL | 1 vial |
| 4 | Blocking Reagent Consisting of: | Buffer that is formulated so as to reduce the non-specific bonds with the surface of the chip and to dilute the anti-Digoxigenin antibody solution. | 20 mL | 1 bottle |
| | Maleic acid 1.2% | | | |
| | NaCl 0.8% | | | |
| | Solid NaOH 0.78% Blocking | | | |
| | reagent(powder) 1% | | | |
| 5 | Detection Buffer Consisting of: | Diluting buffer for the NBT/BCIP chromogenic substrate; it is formulated so as to | 40 mL | 1 bottle |
| | HCL (12N) 0.1% | | | |
| | Tris-base 1.2% | | | |
| | NaCl 0.58% | increase the AP activity. | | |
| | MgCl₂ 1% | | | |
| 6 | NBT/BCIP 100% | Chromogenic substrate. The hydrolysis of BCIP (5-Bromo-4-chloro-3-indolyl phosphate indolyl phosphate), followed by the oxidation of NBT (Nitro blue tetrazolium) in the presence of AP, generates a blue precipitate on the localized zone of enzymatic activity. | 400 µL | 1 vial |
| 9 | BCS HPV Chip | Solid support for the hybridization reaction. Specific probes for each viral genotype, which is the target of the test, adhere to the surface of the support. | 24 Chip | 8 three chip - strips |

### Analytical procedure

### I. DNA Amplification

*1. Preparation of PCR Master Mix.* The reagents, which are shown in Table 6 below, are transferred to a PVC tube and mixed. For each analysis, an additional tube needs to be prepared for the PCR negative and positive control.

**Table 6**

| Reagents | Volume/tube | Number of tubes | Total volume |
|---|---|---|---|
| FM | 39.5 µL | × (n* + 1) | = ... µL |
| DNA Pol. | 0.5 µL | × (n* + 1) | = ... µL |
| 1C | 0.5 µL | × (n* + 1) | = ... µL |

*n = number of samples per test (including negative and positive controls)
2.40 µL of master mix are delivered by means of a pipette into each PCR tube.
3. 10 µL of the extracted DNA are transferred to each tube and are mixed thoroughly; 10 µL of sterile water are added for the negative control and 10 µL of PC (DNA positive control) are added for the positive control. The final volume in each tube is 50 µL.
4. The PCR tubes are located in a thermocycler with heated lid and the following thermal profile is set:
95 °C 3 min 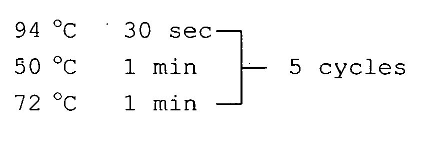 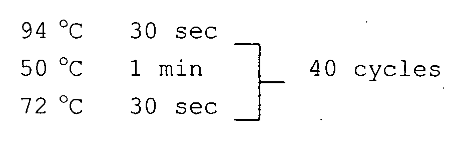
72 °C 5 min

5. At the end of PCR, the hybridization phase is carried out or the amplified products are stored at -20 °C until the use thereof.

### II. Hybridization

### Preparation

1. The BCS Hyb Buffer and the Wash Buffer are taken to ambient temperature.
2. A suitable number of chips (as many as the samples to be tested, plus one for the negative control and one for the positive control) is taken to the same ambient temperature).

### Hybridization reaction

3. 20 µL of the amplified product are delivered by means of a pipette in 180 µL of BCS Hyb Buffer, in a 0.5-ml eppendorff-type tube, and are mixed thoroughly.
4. Denature at 99°C for 5 minutes.
5. Place in ice for another 5 minutes.
6. Each mix is transferred onto Transfer the mix onto a respective chip (BCS HPV Chip); the chips are incubated at 50 °C in a stove for 1 h, being slightly stirred if possible.

### IV. Stringency washing

1. Once the hybridization phase is completed, the liquid is removed from the chip.
2. 250 µL of Wash Buffer are added to the chip, the liquid is well distributed and then it is removed; this washing is repeated a second time.

### V. Blocking

1. A suitable quantity of the antibody anti-Digoxigenin is diluted with the Blocking Reagent, in a 1:2000 ratio (for example: 0.5 µl of antibody in 1 ml of Blocking reagent); 200 µL of this solution are transferred to each chip, which are incubated at ambient temperature, slightly stirred, for 30 minutes. The Blocking Reagent is then removed. 250 µL of Wash Buffer are added to the chip and two washes are carried out.
2. After the second wash, the residual liquid is poured out by upturning each chip on absorbent paper.

### VI. Colorimetric assay

1. 4 µL of NBT/BCIP are mixed with 196 µL of Detection Buffer, and the mixture is transferred onto each chip.
2. The reaction is then continued for 10 minutes in the dark and at ambient temperature.
3. The liquid is then removed, each chip is washed with 250 µL of water and the results are interpreted.

### VII. Interpretation of the results

The results originate from the interpretation of a specific pattern that forms on the chip through the effect of the hybridization reaction and the subsequent colorimetric reaction and owing to the preset arrangement of the probes on the chip. The probe-target hybrids are highlighted by the dark precipitate that is the result of the enzymatic reaction. The chip pattern can be read and interpreted by the naked eye for the > 1 cm² chips, such as those constituting the poly-chip in Figure 1, whilst it is necessary to use an optic reader (BCS Chip Reader) for the analysis of the < 1 cm² chips, such as those comprised in the microplates 30.

The test has been conducted correctly if the zones corresponding to the Hybridization Positive Control and to the PCR Positive Internal Control are evident. If one or more control zones are not visibile on the chip, the test must be repeated.

Figure 2 shows schematically, through symbols, the position of the probes fixed to the surface of the chip that is used in the analytical kit of example 1. In Table 7 below, the meanings of the symbols in Figure 2 are shown.

**Table 7**

| | | | |
|---|---|---|---|
| Cl | Hybridization Control | 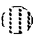 | HPV 16 |
| cp | PCR Internal Control | 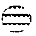 | HPV 18 |
| bg | β-globin | 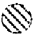 | HPV 26 |
| TA | High-risk types: region E6/E7 | 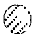 | HPV 30 |
| P | PAN HPV: region E6/E7 | 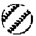 | HPV 31 |
| L | PAN HPV: region L1 | 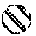 | HPV 33 |
| | | 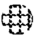 | HPV 35 |
| | | 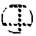 | HPV 39 |
| | | 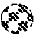 | HPV 45 |
| | | 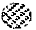 | HPV 51 |
| | | 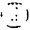 | HPV 52 |
| | | 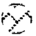 | HPV 53 |
| | | 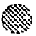 | HPV 56 |
| | | 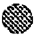 | HPV 58 |
| | | 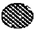 | HPV 59 |
| | | 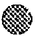 | HPV 66 |
| | | 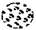 | HPV 67 |
| | | 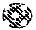 | HPV 68 |
| | | 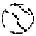 | HPV 69 |
| | | 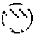 | HPV 70 |
| | | 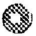 | HPV 73 |
| | | 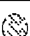 | HPV 82 |

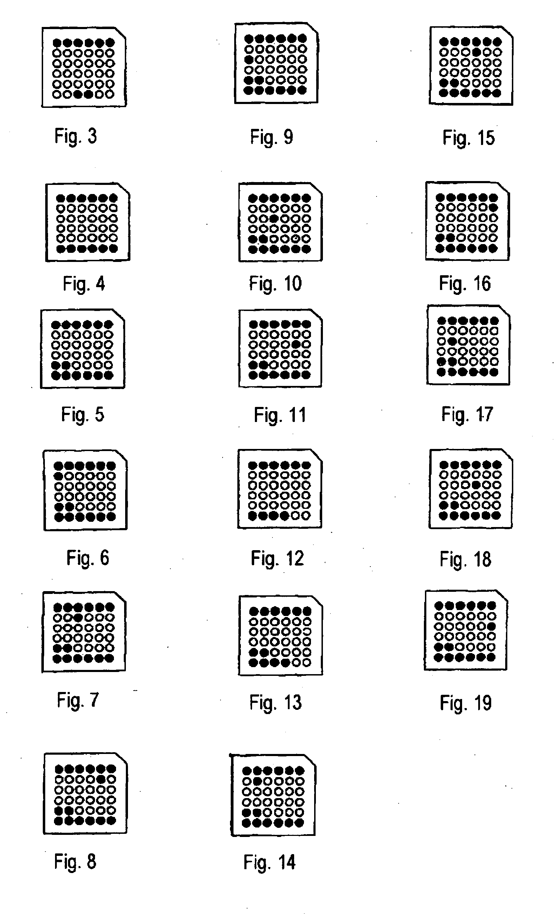

With reference to Figures 3 to 32, examples are shown of patterns and therefore of analytical results obtainable by the chip in Figure 2, depending on the presence or absence in an analysed sample of the various HPV types that are identifiable by the kit.

Figure 3 shows a negative sample.

Figure 4 shows a positive sample for low-risk HPV types.

Figure 5 shows a positive sample for high-risk HPV types, not comprised amongst the typable ones.

Figure 6 shows a positive sample for HPV 16.

Figure 7 shows a positive sample for HPV 26.

Figure 8 shows a positive sample for HPV 31.

Figure 9 shows a positive sample for HPV 35.

Figure 10 shows a positive sample for HPV 45.

Figure 11 shows a positive sample for HPV 52.

Figure 12 shows a positive sample for low-risk HPV types, with deletion in L1.

Figure 13 shows a positive sample for high-risk HPV types, not comprised amongst the typable ones, with deletion in L1.

Figure 14 shows a positive sample for HPV 18.

Figure 15 shows a positive sample for HPV 30.

Figure 16 shows a positive sample for HPV 33.

Figure 17 shows a positive sample for HPV 39.

Figure 18 shows a positive sample for HPV 51.

Figure 19 shows a positive sample for HPV 53.

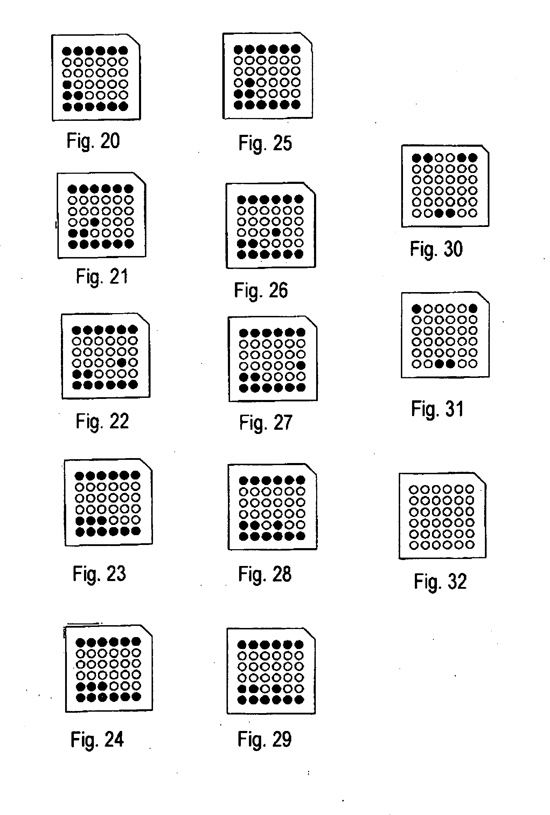

Figure 20 shows a positive sample for HPV 56.

Figure 21 shows a positive sample for HPV 59.

Figure 22 shows a positive sample for HPV 67.

Figure 23 shows a positive sample for HPV 69.

Figure 24 shows a positive sample for HPV 73.

Figure 25 shows a positive sample for HPV 58.

Figure 26 shows a positive sample for HPV 66.

Figure 27 shows a positive sample for HPV 68.

Figure 28 shows a positive sample for HPV 70.

Figure 29 shows a positive sample for HPV 82.

Figure 30 shows a sample in which the control for β-globin is absent, since the extraction procedure did not enable a suitable quantity of nucleic acid to be obtained.

Figure 31 shows a sample in which the PCR internal positive control is absent, since the extraction procedure did not enable a suitable nucleic acid to be obtained, for example due to the presence of possible inhibitors.

Figure 32 shows a sample in which hybridization internal controls are not present as some problem has affected the analysis also during the detection phase.

In the case of analytical results like those shown in Figures 30-32, the analysis has to be repeated.

To assess the performance of the above disclosed kit, the nucleic acid extracted from frozen vaginal spatula samples and frozen or paraffin-included biopsies has been parallely analysed using BCS HPV CHIP and ProDect HPV (the operation of which is described in the above-mentioned WO0185994) as a reference test. The data emerging from this experimental comparison are set out in Table 8 below.

**Table 8**

| Regions | Number of samples (neg. + posit.) | Diagnostic specificity | Diagnostic Sensitivity | Analytical sensitivity Copies/PCR |
|---|---|---|---|---|
| L1 | 30 | 96% | 98.5% | 10⁻² |
| E6/E7 high-risk | 30 | 99.4 % | 97.4 % | 10³ |
| E6/E7 low-risk | 30 | 99.2 % | 96.3 % | 10³ |

### Example 2

The method according to the invention has been applied, in the form of an analytical kit, to detect the following enteric viruses in food samples: *Enterovirus, EV71, CoxsA16, HAV, HEV, Norwalk Virus, Rotavirus, Adenovirus.* These viruses, which are aetiological agents of gastroenteritis, are transmittable via water and foodstuffs, in particular fish products, and are also detectable in the faecal material of the infected persons. The composition of the kit is similar to what has been previously disclosed in example 1, differing from the latter mainly by the sequences of primers and probes and by the PCR thermal profile. In fact, for certain viruses it is necessary to reverse-transcribe the genomic nucleic acid (RNA) in a complementary DNA filament. Part A of the kit is thus composed as set out below in Table 9 and comprises an additional mix containing the oligonucleotides required for the reverse transcription. Part B of the kit is composed like the part B disclosed in the preceding example 1. The kit does not comprise: the reagents for the extraction of the nucleic acid, the reagents for the reverse-transcription phase of the nucleic acid RNA in a complementary DNA and the "Ready-To-Go RT-PCR Beads" (Amersham Biosciences, 27-9266-01). With the exception of the presence of the reverse-transcription phase, the analytical procedure provides phases that are similar to those disclosed in the preceding example 1.

**Table 9**

| **N°** | **Solution** | **Function** | **Volume** | **Quantity** |
|---|---|---|---|---|
| 1 | **RT Primers** | RTPCR premix, for reverse transcribing specific sequences of viral RNA of *Enterovirus, EV71, CoxsA16, HAV, HEV, Norwalk Virus, Rotavirus, Adenovirus;* the premix has to be used together with the "Ready-To-Go RT-PCR Bead" dissolved in water. | | |
| | Consisting of Oligonucleotides 0.5% : | | | |
| | biotm-oligo F7 (100µM) | | | |
| | biotin-oligo R4 (100µM) | | | |
| | biotin-HAV1 (100 µM) | | | |
| | biotin-HAV2 (100 µM) | | | |
| | biotin-NV1 (100 µM) | | | |
| | biotin-NV2 (100 µM) | | 0.4 ml | |
| | biotin-RVA (100 µM) | | | |
| | biotin-RVB (100 µM) | | | 1 vial |
| | biotin-HEVR473(100 µM) | | | |
| | biotin-HEV51 (100 µM) | | | |
| 2 | **EV Mix** | PCR premix for amplifying specific sequences of DNA_{c} and *Enterovirus, EV71, CoxsA16, HAV, HEV, Norwalk Virus, Rotavirus, Adenovirus* and an internal control DNA; the premix consists of 5'biotinylated primers, deoxyrobonucleotides (dNTPs), PCR buffer, MgCl₂ and water. | | |
| | Consisting of: | | | |
| | -DNTP 2.5 mM 1.3% | | | |
| | -10xPCR Buffer 15% | | | |
| | -MgCl₂ (25mM) 12.67% | | | |
| | -Oligonucleotides 5% : | | | |
| | biotm-oligo F7 (100µM) | | | |
| | biotin-oligo R4 (100µM) | | | |
| | biotin-HAV1 (100 µM) | | | |
| | biotin-HAV2 (100 µM) | | | |
| | biotin-NV1 (100 µM) | | | |
| | biotin-NV2 (100 µM) | | | |
| | biotin-RVA (100 µM) | | | 1 vial |
| | biotin-RVB (100 µM) | | | |
| | biotin-HEVR473 (100 µM) | | 1.4 mL | |
| | biotin-HEV51 (100 µM) | | | |
| | biotin-advfl (100 µM) | | | |
| | biotin-advrl (100 µM) | | | |
| | biotin-R1 (100 µM) | | | |
| | biotin-F5 (100 µM) | | | |
| | Actin-DNA 8.33% | | | |
| 3 | **DNA Polymerase** 100% | DNA Polymerase Enzyme (5U/µL). | 24 µL | 1 vial |
| 4 | **DEPC Water** 100% | Diluent for "Ready-To-Go RT-PCR Bead" | 1.3 ml | 1 tube |

Figure 33 shows schematically, through symbols, the position of the probes fixed to the surface of the chip that is used in the analytical kit in example 2. Table 10, set out below, shows the meanings of the symbols in Figure 33.

**Table 10**

| | | | |
|---|---|---|---|
| CI | Positive hybridization control | **EV** | EV71 |
| CP | PCR Positive Control | **CX** | CoxsA16 |
| N | Norwalks Virus | **HA** | HAV |
| R | Rotavirus | **HE** | HEV |
| A | Adenovirus | | |
| PE | Pan Enterovirus | | |

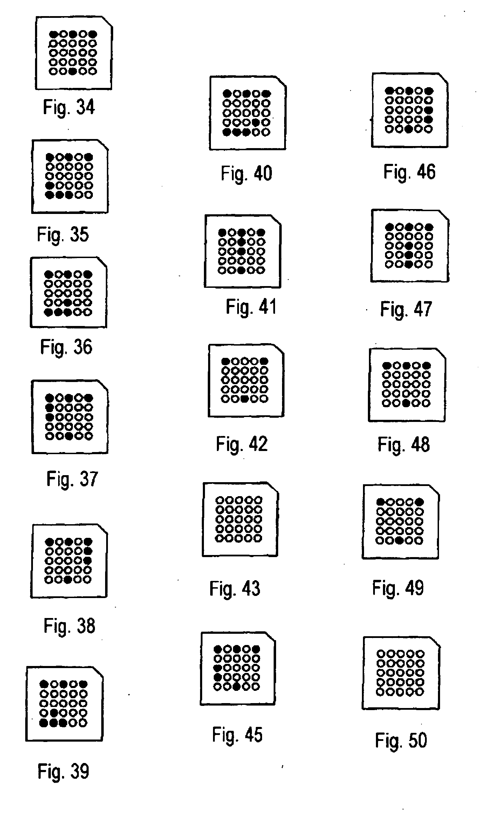

With reference to Figures 34 to 43, examples are shown of patterns, and therefore of analytical results, obtainable by means of the chip in Figure 33, according to the presence or absence in an analysed sample of the various enteric viruses that are identifiable by means of the kit.

Figure 34 shows a negative sample.

Figure 35 shows a positive sample for EV71.

Figure 36 shows a positive sample for HAV.

Figure 37 shows a positive sample for the Norwalks virus.

Figure 38 shows a positive sample for Adenovirus.

Figure 39 shows a positive sample for Coxsachievirus A16.

Figure 40 shows a positive sample for HEV.

Figure 41 shows a positive sample for Rotavirus.

Figure 42 shows a sample in which the PCR internal negative control is absent, since the extraction procedure has not enabled a suitable nucleic acid to be obtained, for example due to the presence of possible inhibitors.

Figure 43 shows a sample in which hybridization internal controls are not present as some problem has affected the analysis also during the detection phase.

In the case of analytical results like those in Figures 42 and 43, the analysis has to be repeated.

Tables 11-13 below show the sequences of the probes and of the primers that are used and the amplified genomic regions of the pathogenic viruses searched for.

**Table 11 (probes)**

| | | | mer |
|---|---|---|---|
| 1 | VP1 | ATGAAGCAYGTCAGGGCRTGGATACCTCG | 29 |
| 2 | Probe1 | TCCTCCGGCCCCTGAATGCGGCTAATC | 27 |
| 3 | Probe2 | TGTCGTAACGSGCAASTCYGYRGCGGAACCGAC | 33 |
| 4 | Probe3 | TACTTTGGGTGTCCGTGTTTCHTTTTAT | 28 |
| 5 | EV 71-2 | CTTATAAGCAGACTCAACCCGGTGCTGATG | 30 |
| 6 | EV 71-3 | TGGCATTCCAATATCACAATTAACAGTG | 28 |
| 7 | EV71-6-1 | RCARCACCCGTACGTGCTYGATGCTGGSA | 29 |
| 8 | CoxA16-1 | CTCGGCACTATCGCAGGAGGGACCGGGAAT | 30 |
| 9 | Cox 16-2 | CCTACGCCACTACACAGCCTGGTCAGGTTG | 30 |
| 10 | ADV-P4 | TAACATgACCAARgACTggTTCCTggT | 27 |
| 11 | ADV-P5 | ACAAYATYggMTACCAgggCTTYTA | 25 |
| 12 | ADV-P7 | AACATgACCAAggACTggTTCCTggT | 25 |
| 13 | ADV-P8 | ACAATATTggCTACCAgggCTTCTA | 25 |
| 14 | actin | GAGCCGGAAATCGTGCGCGACATCAAGGAG | 30 |
| 15 | HAV | CTCCAGAATCATCTCC | 16 |
| 16 | NV | CTGCCATCTGGATTCCCATGTA | 22 |
| 17 | RV1 | GTATGGTATTGAATATACCAC | 21 |
| 18 | RV2 | GTCACCATCATTGATTGAGTACTT | 24 |
| 19 | RV3 | TTCATCATCTGAAATCTCATTTTTA | 24 |
| 20 | RV4 | TGAATTATCATTTATTTCTGTTGCT | 24 |
| 21 | RV4 | TTCGTGTCACTAATTTGAGTTGGA | 24 |
| 22 | HEVR383 | TACCAACGCTGAACATCACG | 20 |

**Table 12 (primers)**

| | | | |
|---|---|---|---|
| 1 | F7 | biotin-GGITGGTRSTGGAARTTICC | 20 |
| 2 | R4 | biotin-ARRTTTATCCAYTGRTGIGG | 20 |
| 3 | HAV1 | biotin-GGAAATGTCTCAGGTACTTTCTTTG | 25 |
| 4 | HAV2 | biotin-GTTTTGCTCCTCTTTATCATGCTATG | 26 |
| 5 | NV1 | biotin-CTTGTTGGTTTGAGGCCATAT | 21 |
| 6 | NV2 | biotin-ATAAAAGTTGGCATGAACA | 19 |
| 7 | RVA | biotin-GGCTTTAAAAGAGAGAATTTCCGTCTGG | 28 |
| 8 | RVB | biotin-GATCCTGTTGGCCATCC | 17 |
| 9 | R1 actina | biotin-CACYGGATGGCCAATCCAA | 19 |
| 10 | F5 actina | biotin-AAGAGYCTATTGAGCTA | 17 |
| 11 | adv-f1 | biotin-CCACCTTCTTCCCCAT | 16 |
| 12 | adv-r1 | biotin-CTCATKggCTggAAgTT | 17 |
| 13 | HEVR473 | biotin-CCTCGAAGCAGTAAGTGCGGTC | 22 |
| 14 | HEV51 | biotin-GGCTCCTGGCATCACTACTG | 20 |

| | | | |
|---|---|---|---|
| (Wherein : Y=C,T ; R=A,G ; H=A, T, C ; M=A, C; K=G,T) | | | |

**Table 13 (Amplified viral regions)**

| Target | Name of the Primer | Sequence PCR 5' → 3' |
|---|---|---|
| PanEV NCBI Reference sequences: see Annex 1 | F5 | |
| | R1 | |
| | Length: 223 pb | |
| EV 71 NCBI Reference sequences: see Annex 2 | F7 | |
| | R4 | |
| | Length: 326 pb | |
| CA 16 NCBI Reference sequence:AF177 911 | F7 | |
| | R4 | |
| | Length: 236 pb | |
| Adenovirus NCBI Reference sequence:AF177 911 | Adv-f1 | |
| | Adv-r1 | |
| | Length: 556 pb | |
| Actin313 NCBI Reference sequence:Gi501 6088 | F5 | |
| | R1 | |
| | Length: 313 pb | |
| HEV NCBI Reference sequence: Gi1209363 | HEVR473 | |
| | HEV51 | |
| | Length: 420 pb | |
| HAV NCBI Reference sequence: G18810242 | HAV1 | |
| | HAV2 | |
| | Length: 246 pb | |
| NV NCBI Reference sequence: Gi1061311 | NV1 | |
| | NV2 | |
| | Length: 470 pb | |
| RV Reference sequence NCBI Gi22652625 | RVA | |
| | RVB | |
| | Length: 392 pb | |

The NCBI reference sequences relating to the targets Pan EV and EV 71 are set out below, in Annex 1 and in Annex 2:

### Annex 1

gi|458298|gb|U05876.1|CAU05876 "Coxsackievirus A16 G-10, complete genome" 442 e-123;

gi|59045|emb|X67706.1|CXB5CGA "Coxsackievirus B5 complete genome" 394 e-109;

gi|42662142|db|AB162739.1 "Human coxsackievirus A4 gene for polyprotein, partial cds, isolate:2319/CA4/Kanagawa/2003" 379 e-104;

gi|42662140|dbj|AB162738.1 "Human coxsackievirus A2 gene for polyprotein, partial cds, isolate: P-2329/CA2/Kanagawa/2003" 379 e-104;

gi|38347774|dbj|AB126200.1 "Human coxsackievirus A4 gene for polyprotein, partial cds,isolate:P-2232/CA4/Kanagawa/2003" 379 e-104;

gi|38347770|dbj|AB126198.1 "Human coxsackievirus A2 gene for polyprotein, partial cds, isolate:P-2202/CA2/Kanagawa/2003" 379 e-104;

gi|14139982|emb|AJ295194.1|HCO295194 "Human coxsackievirus B3 genomic RNA for partial polyprotein gene, isolate VR-30, ATCC" 379 e-104;

gi|14139980|emb|AJ295193.1|HCO295193 "Human coxsackievirus B B1 genomic RNA for partial polyprotein gene, isolate VR-687, ATCC" 379 e-104;

gi|323427|gb|M21949.1|CXCAP "Coxsackievirus B3 capsid/processing polyprotein gene, 5' end" 379 e-10; gi|323432|gb|M88483.1|CXAB3CG "Coxsackievirus B3 mRNA, complete genome" 379 e-104;

gi|34485421|gb|AY302541.1| "Human echovirus 15 strain CH 96-51, complete genome" 379 e-104;

gi|323421|gb|M16572.1|CXA3G "Coxsackievirus B3, complete genome" 379 e-104;

gi|38347772|dbj|AB126199.11 "Human coxsackievirus A2 gene for polyprotein, partial cds, isolate: P-2242/CA2/Kanagawa/2003" 373 e-102;

gi|3234191gb|M33854.1|CXA3CG "Coxsackievirus B3 (CVB3) complete genome" 371 e-102;

gi|10863164|gb|AF231765.1|AF231765 "Coxsackievirus B3 strain PD, complete genome." 371 e-102;

gi|10863162|gb|AF231764.1|AF231764 "Coxsackievirus B3 strain P, complete genome" 371 e-102;

gi|10863160|gb|AF231763.1|AF231763 "Coxsackievirus B3 strain 31-1-93, complete genome" 371 e-102;

gi|323417|gb|M16560.1|CXA1G "Coxsackievirus B1, complete genome" 371 e-102;

gi|38347776|dbj|AB126201.1| "Human coxsackievirus A5 gene for polyprotein, partial cds, isolate:P-2201/CA5/Kanagawa/2003" 363 2e-99;

gi|38347768|dbj|AB126197.1| "Human coxsackievirus A2 gene for polyprotein, partial cds, isolate:P-2181/CA2/Kanagawa/2003" 363

2e-99;

gi|323429|gb|M74567.1|CXAMYOJ "Coxsackievirus B3 gene, 5' flank" 363 2e-9;

gi|34485451|gb|AY302556.11 "Human echovirus 33 strain Toluca-3 complete genome" 363 2e-99;

gi|344854311gblAY302546.11 "Human echovirus 20 strain JV-1 complete genome" 363 2e-99;

gi!12584545|emb|AJ304423.1|HCO304423 "Human coxsackievirus B4 genomic RNA for partial polyprotein (VP4,VP2), isolate ITA94-A2BGM" 355 5e-97;

gi|38347778|dbj|AB126202.1| "Human coxsackievirus A5 gene for polyprotein, partial cds,isolate:P-2215/CA5/Kanagawa/2003" 355 5e-97;

gi|28610113|gb|AF524866.1| "Human echovirus 9 strain Barty, complete genome" 355 5e-97;

gi|23096014|dbj|AB081380.1| "Human enterovirus 71 gene for polyprotein, partial cds,isolate:V-14457/EV71/Kanagawa/1997" 355 5e-97;

gi|16117820|dbj|AB059826.1| "Enterovirus 71 RNA, 5'UTR, partial sequence, strain:SK-EV006/Malaysia 97" 355 5e-97;

gi|34485423|gb|AY302542.1| "Human echovirus 16 strain Harrington complete genome" 355 5e-97;

gi|12584555|emb|AJ304428.1|HCO304428 "Human coxsackievirus BB3 genomic RNA for partial polyprotein (VP4,VP2), isolate ITA94-A5HEP" 347 1e-94;

gi|12584551|emb|AJ304426.1|HCO304426 "Human coxsackievirus BB3 genomic RNA for partial polyprotein (VP4,VP2), isolate ITA94-B6HEP" 347 1e-94;

gi|12584547|emb|AJ304424.1|HCO304424 "Human coxsackievirus BB3 genomic RNA for partial polyprotein (VP4,VP2), isolate ITA94-H3HEP" 347 1e-94;

gi|12584541|emb|AJ304421.1|HCO304421 "Human coxsackievirus BB3 genomic RNA for partial polyprotein (VP4,VP2), isolate ITA94-A3BGM" 347 1e-94;

gi|38347786|dbj|AB126206.1| "Human coxsackievirus A10 gene for polyprotein, partial cds, isolate:P-2211/CA10/Kanagawa/2003" 347 1e-94;

gi|38347780|dbj|AB126203.1| "Human coxsackievirus A6 gene for polyprotein, partial cds, isolate:P-2220/CA6/Kanagawa/2003" 347 1e-94;

gi|141429994|emb|AJ295200.1|HCO295200 "Human coxsackievirus A9B genomic RNA for partial polyprotein gene,isolate VR-186, ATCC" 347 1e-94;

g|123096016|dbj|AB081381.1| "Human enterovirus 71 gene for p71 gene for polyprotein, partial cds, isolate: V-14570/EV71/Kanagawa/1997" 347 1e-94;

gi|23095988|dbj|AB081367.1| "Human enterovirus 71 gene for polyprotein, partial cds,isolate: V-9281/EV71/Kanagawa/1990" 347 1e-94;

gi|1171120|gb|U22521.1|ETU22521 "Human enterovirus 71 polyprotein gene, complete cds" 347 1e-94;

gi|7716639|gb|AF251358.1|AF251358 "Enterovirus 71 strain 13/Sin /98 polyprotein mRNA, partial cds" 347 1e-94; gi|1373227|gb|U57056.1|CXU57056 "Coxsackievirds B3 Woodruff variant, complete genome" 347 1e-94;

gi|221214|dbj|D00627.1|CXA9CG "Coxsackievirus A9 genomic RNA, complete genome, strain: Griggs" 347 1e-94;

gi|34485441|gb|AY302551.| "Human echovirus 27 strain Bacon complete genome" 347 1e-94;

gi|42662160|dbj|AB162748.1| "Human coxsackievirus B1 gene for polyprotein, partial cds, isolate: HO792csf/CB1/Kanagawa/2003" 345 5e-94;

gi|42558240|dbj|AB126216.2| "Human coxsackievirus B1 gene for polyprotein, partial cds,isolate:HC72csf/CB1/Kanagawa/2003" 345 5e-94;

gi|38347798|dbj|AB126212.1| "Human coxsackievirus B1 gene for polyprotein, partial cds, isolate: P-2208/CB1/Kanagawa/2003" 345 5e-94;

gi|1199448|gb|L76400.1|ECH5UTRE "Echovirus type 7 2185/Finland/87 5' UTR sequence" 341 7e-93;

gi|28610115|gb|AF524867.1| "Human echovirus. 9 isolate DM, complete genome" 339 3e-92 ;

gi|14139996|emb|AJ295201.1|HCO295201 "Human coxsackievirus A9 genomic RNA for partial polyprotein gene,isolate VR-1311, ATCC" 339 3e-92 ;

gi|4581992|emb|AJ133661.1|E30133661 "Echovirus type 30 partial ORF1 gene for polyprotein, isolate 89T2090" 33.9 3e-92 ; gi|27657694|gb|AY167105.1| "Human echovirus 11 strain Kust/86 polyprotein gene, complete cds" 339 3e-92;

gi|27657690|gb|AY167103.1| "Human echovirus 11 strain Hun/90 polyprotein gene, complete cds" 339 3e-92;

gi|18158536|gb|AY055173.1| "Human enterovirus 71 isolate 236-TW-86 5' untranslated region, partial sequence" 339 3e-92;

gi|18158517|gb|AY055154.1 "Human enterovirus 71 isolate N5101-TW-98 5' untranslated region, partial sequence" 339 3e-92;

gi|18158516|gb|AY055153.1| "Human enterovirus 71 isolate N7129-TW-99 5' untranslated region, partial sequence" 339 3e-92;

gi|18158515|gb|AY055152.1| "Human enterovirus 71 isolate N7008-TW-99 5' untranslated region, partial sequence" 339 3e-92;

gi|17224874|gb|AF311939.1|AF311939 "Human coxsackievirus B4 strain E2 variant, complete genome" 339 3e-92;

gi|5881228|gb|AF177911.1|AF177911 "Coxsackievirus A16 polyprotein gene, complete cds" 339 3e-92;

gi|23096012|dbj|AB081379.1| "Human enterovirus 71 gene for polyprotein, partial cds, isolate:V-14433/EV71/Kanagawa/1997" 339 3e-92;

gi|23096010|dbj|AB081378.1| "Human enterovirus 71 gene for polyprotein, partial cds, isolate: V-14429/EV71/Kanagawa/1997" 339 3e-92;

gi|23095942|dbj|AB081344.1| "Human enterovirus 71 gene for polyprotein, partial cds, isolate: V-4083/EV71/Kanagawa/1983" 339 3e-92;

gi|1171122|gb|U22522.1|ETU22522 "Human enterovirus 71 polyprotein gene, complete cds" 339 3e-92;

gi|13128902|gb|AF241359.1|AF241359 "Enterovirus CA55-1988, complete genome" 339 3e-92;

gi|7716641|gb|AF251359.1|AF251359 "Enterovirus 71 * strain 18/Sin/97 polyprotein mRNA, partial cds" 339 3e-92;

gi|914055|gb|S76772.1|S76772 "polyprotein [Coxsackie B4 virus CB4, host = mice, E2, originally derived from Edwards CB4 human strain, Genomic RNA Complete, 7397 nt]" 339 3e-92;

gi|16117819|dbj|AB059825.1| "Enterovirus 71 RNA, 5'UTR, partial sequence, strain: C7/Osaka/Japan 97" 339 3e-92;

gi|16117814|dbj|AB059820.1| "Enterovirus 71 RNA, 5'UTR, partial sequence, strain: Nagoya/Japan 73" 339 3e-92;

gi|34485443|gb|AY302552.1| "Human echovirus 29 strain JV-10 complete genome" 339 3e-92;

gi|5670265|gb|AF167996.1|AF167996 "Enterovirus 71 strain TW/253/86 polyprotein gene, partial cds; and 5' untranslated region, partial sequence" 339 3e-92;

gi|38347802|dbj|AB126214.1| "Human coxsackievirus B1 gene for polyprotein, partial cds, isolate: P-2240/CB1/Kanagawa/2003" 337 1e-91;

gi|38347796|dbj|AB126211.1| "Human coxsackievirus B1 gene for polyprotein, partial cds, isolate: P-2199/CB1/Kanagawa/2003" 337 1e-91;

gi|14139944|emb|AJ295173.1|HEC295173 "Human echovirus 30 genomic RNA for partial 5'UTR, isolate ITA98-08" 335 5e-91;

gi|14139940|emb|AJ295171.1|HEC295171 "Human echovirus 30 genomic RNA for partial polyprotein gene, isolate ITA97-05" 333 2e-90;

gi|23095946|dbj|AB081346.1| "Human enterovirus 71 gene for polyprotein, partial cds, isolate: V-4185/EV71/Kanagawa/1983" 333 2e-90;

gi|23095944|dbj|AB081345.1| "Human enterovirus 71 gene for polyprotein, partial cds, isolate: V-4110/EV71/Kanagawa/1983" 333 2e-90;

gi|12584537|emb|AJ304419.1|HCO304419 "Human coxsackievirus B3 genomic RNA for partial polyprotein (VP4,VP2), isolate ITA94-B4HEP" 331 7e-90;

gi|42662150|dbj|AB162743.1| "Human coxsackievirus A10 gene for polyprotein, partial cds, isolate: P-2267/CA10/Kanagawa/2003" 331 7e-90;

gi|4572558|emb|X90722.2|EV25JV4 "Human echovirus 25 mRNA for partial polyprotein, isolate JV-4" 331 7e-90;

gi|4581994|emb|AJ133662.1|E30133662 "Echovirus type 30 partial ORF1 gene for polyprotein, isolate 91TLC" 331 7e-9;

gi|27657692|gb|AY167104.1| "Human echovirus 11 strain Kar/87 polyprotein gene, complete cds" 331 7e-9;

gi|18158533|gb|AY055170.1| "Human enterovirus 71 isolate S0296-TW-00 5' untranslated region, partial sequence" 331 7e-90;

gi|7408183|gb|AF029859.2|AF029859 "Echovirus 1 (strain Farouk /ATCC VR-1038) polyprotein precursor, gene, complete cds" 331 7e-90;

gi|23095982|dbj|AB081364.1| "Human enterovirus 71 gene for polyprotein, partial cds, isolate: V-7949/EV71/Kanagawa/1988" 331 7e-90;

gi|23095940|dbj|AB081343.1| "Human enterovirus 71 gene for polyprotein, partial cds, isolate: V-4014/EV71/Kanagawa/1983" 331 7e-90;

gi|16117816|dbj|AB059822.11 "Enterovirus 71 RNA, 5'UTR, partial sequence, strain: Hungary 78" 331 7e-9;

gi|16117815|dbj|AB059821.1| "Enterovirus 71 RNA, 5'UTR, partial sequence, strain: 258/Bulgaria" 331 7e-90;

gi|34485437|gb|AY302549.1| "Human echovirus 25 strain JV-4 complete genome" 331 7e-90;

gi|38347800|dbj|AB126213.1| "Human coxsackievirus B1 gene for polyprotein, partial cds, isolate: P-2233/CB1/Kanagawa/2003" 329 3e-89;

gi|37993797|gb|AY429470.1 "Swine vesicular disease virus strain HK'70, complete genome" 327 1e-88;

gi|61167|emb|X54521.1|PISVDV "Swine vesicular disease virus complete genomic RNA" 327 1e-88;

gi|18158528|gb|AY055165.1| "Human enterovirus 71 isolate M0485-TW-00 5' untranslated region, partial sequence" 327 1e-88;

gi|402536|dbj|D16364.1|SVDMPS "Swine vesicular disease virus gene for polyprotein, complete cds" 327 1e-88;

gi|26006300|gb|AF328683.1| "Human coxsackievirus B4 polyprotein-like gene, complete sequence" 325 4e-88;

gi|23095980|dbj|AB081363.1| "Human enterovirus 71 gene for polyprotein, partial cds, isolate: V-6458/EV71/Kanagawa/1986" 325 4e-88;

gi|23095978|dbj|AB081362.1| "Human enterovirus 71 gene for polyprotein, partial cds, isolate: V-6408/EV71/Kanagawa/1986." 325 4e-88;

gi|23095950|dbj|AB081348.1| "Human enterovirus 71 gene for polyprotein, partial cds,isolate: V-6118/EV71/Kanagawa/1986" 325 4e-88;

gi|37623503|gb|AY342850.1| "Human coxsackievirus B5 patient BE99-9967 5' UTR, partial sequence" 325 4e-88;

gi|42662172|dbj|AB162754.1| "Human coxsackievirus B2 gene for polyprotein, partial cds, isolate: HO312f/CB2/Kanagawa/2002" 323 2e-87;

gi|42662168|dbj|AB162752.1| "Human coxsackievirus B2 gene for polyprotein, partial cds, isolate: P-1850/CB2/Kanagawa/2002" 323 2e-87;

gi|42662166|dbj|AB162751.1| "Human coxsackievirus B2 gene for polyprotein, partial cds, isolate: P-1832/CB2/Kanagawa/2002" 323 2e-87

(Altschul, Stephen F., Thomas L. Madden, Alejandro A. Schäffer, Jinghui Zhang, Zheng Zhang, Webb Miller, and David J. Lipman (1997) "Gapped BLAST and PSI-BLAST: a new generation of protein database search programs", Nucleic AcidsRes. 25:3389-3402).

### Annex 2

gi|1171120|gb|U22521.1|ETU22521 "Human enterovirus polyprotein gene, complete cds" 646 0.0;

gi|10799098|gb|AF302996.1|AF302996 "Enterovirus 71 strain SHZH98 polyprotein gene, complete cds" 236 2e-59;

gi|1171122|gb|U22522.1|ETU22522 "Human enterovirus 71 polyprotein gene, complete cds" 222 3e-55

(Altschul, Stephen F., Thomas L. Madden, Alejandro A. Schäffer, Jinghui Zhang, Zheng Zhang, Webb Miller, and David J. Lipman (1997) "Gapped BLAST and PSI-BLAST: a new generation of protein database search programs", Nucleic AcidsRes. 25:3389-3402).

To assess the performance of the kit, supernatants of cell cultures were used that were infected with different isolates or strains of the viruses indicated in Table 14 below, which shows the experimentally obtained results in terms of sensitivity and specificity:

**Table 14**

| Target pathogens | Unit: PFU/ ml | |
|---|---|---|
| | Sensitivity | Specificity |
| PanEnterovirus | 10³ | 95% |
| EV71 | 10² | 98% |
| CoxsA16 | 10² | 99% |
| HAV | 10² | 97% |
| HEV | 10² | 92% |
| Norwalk Virus | 10² | 94% |
| Rotavirus | 10³ | 93% |
| Adenovirus | 10² | 96% |

The analytical sensitivity of the method was evaluated through a comparison with the plaque reduction test and was expressed in PFU/ml (Colony-Forming Units/ml). 200 µl of scaled dilutions (ratio 1:10) of the supernatant containing the single viruses were titrated on single layers of permissive cells on a medium to which 1% carboxy-methyl cellulose or 2% agarose had been added. After 24-96 hours of incubation at 37°C in 5% CO₂, the single layer of cells was fixed with a 10% ethanol-acetic acid solution and was then stained with of a 1% trypan blue solution. Since a virus that infects a cell in these conditions is able only to infect the cells that are contiguous to the latter, each area in which the cellular lysis occurs and which is consequently non-stainable, (so-called "plaque") corresponds to an infecting virus that was originally present in the inoculum. From another 200 µl of the same viral dilutions the nucleic acid was extracted by means of a kit on silica column (Roche, High Pure Nucleic Acid Kit). 100 µl of the viral stocks having different titration were mixed together (10⁶-10⁴-10² PFU/ml), in order to evaluate any interference and/or reductions in sensitivity that may occur when several genic targets are present in the original sample. No interference or reduction of sensitivity was detected.

### Example 3

The method according to the invention has been applied, in the form of an analytical kit, to searching for and identifying in biological samples, for example expectorations, certain bacteria that are aetiological agents of acute respiratory syndromes in humans and are therefore highly transmittable by air, namely *Chlamydia pneumoniae, Legionella pneumophila* and *Mycoplasma pneumoniae.* The kit is particularly intended for conducting a prompt differentiating diagnosis between the aforementioned pathogenic agents.

The composition of the kit is similar to what has been previously disclosed in example 2, differing from the latter only by the sequences of primers and probes and by the amplification reaction. In fact, as the pathogens searched for are DNA bacteria, reverse-transcribing of the the nucleic acid is not necessary. Part A of the kit thus has the composition illustrated in Table 15, set out below, and comprises a single mix containing the oligonucleotides and all the reagents required for amplification. Part B of the kit is composed like the part B disclosed in the preceding example 1. The kit does not comprise the reagents for extracting the nucleic acid. The analytical procedure provides similar phases to those disclosed in the preceding example 1.

**Table 15**

| **N°** | **Solution** | **Function** | **Volume** | **Quantity** |
|---|---|---|---|---|
| 1 | **RB Mix** | PCR premix for amplifying specific sequences of *Chlamydia pneumoniae, Legionella pneumophila e Mycoplasma pneumoniae* and the internal control DNA; the premix consists of 5'biotinylated primers, deoxyrobonucleotides (dNTPs), PCR buffer, MgCl₂ and water. | | |
| | Consisting of: | | | |
| | -DNTP 2.5 mM 1.3% | | | |
| | -10xPCR Buffer 15% | | | |
| | -MgCl₂ (25mM) 12.67% | | | |
| | -Oligonucleotides 5% : | | | |
| | Biotin-CPF (100 pM) | | | |
| | Biotin- CPR (100 µM) | | 1.4 mL | 1 vial |
| | Biotin- MPF (100 µM) | | | |
| | Biotin- MPR (100 µM) | | | |
| | Biotin- LPmipAf(100µM) | | | |
| | Biotin- LPmipAr(100µM) | | | |
| | Biotin-R1 (100 µM) | | | |
| | Biotin-F5 (100 µM) | | | |
| | Actina-DNA 8.33% | | | |
| 2 | **DNA Polymerase** 100% | DNA Polymerase Enzyme (5U/µL). | 24 µL | 1 vial |

Figure 44 schematically shows, through symbols, the positions of the probes fixed to the surface of the chip that is used in the analytical kit of example 3. Table 16, set out below, shows the meanings of the symbols in Figure 44.

**Table 16**

| | |
|---|---|
| CI | Positive Hybridization control |
| CP | PCR Positive Control |
| PN | C.pneumoniae |
| MP | M.pneumoniae |
| LP | L.pneumophila |

With reference to Figures 45 to 50, examples are shown of patterns and therefore of analytical results obtainable by means of the chip in Figure 44, according to the presence or absence in an analysed sample of the different types of pathogen bacteria identifiable by the kit.

Figure 45 shows a positive sample for Chlamydia pneumoniae.

Figure 46 shows a positive sample for Legionella pneumophila.

Figure 47 shows a positive sample for Mycoplasma pneumoniae.

Figure 48 shows a negative sample.

Figure 49 shows a sample in which the PCR internal negative control was absent, since the extraction procedure did not allow a suitable nucleic acid to be obtained, for example due to the presence of possible inhibitors.

Figure 50 shows a sample in which the hybridization internal controls are not present, since some problem has affected the analysis also during the detection phase.

In the case of analytical results like the one in Figures 49 and 50, the analysis has to be repeated.

Tables 17-19, set out below, show the sequences of probes and primers used and the amplified genic sequences of the pathogen bacteria searched for.

**Table 17 (primers)**

| N° | NAME | Sequence(s) 5' → 3' | mer |
|---|---|---|---|
| 1 | CPF | Biotin-GGTCTCAACCCCATCCGTGTCGG | 23 |
| 2 | CPR | Biotin-TGCGGAAAGCTGTATTTCTACAGTT | 25 |
| 3 | MPF | Biotin-CCCTCGACCAAGCCAACCTC | 20 |
| 4 | MPR | Biotin-TGCGCGTTGTTCTTGTTGGTG | 21 |
| 5 | LPmipAf | Biotin-ACCGAACAGCAAATGAAAGA | 20 |
| 6 | LPmipAr | Biotin-AACGCCTGGCTTGTTTTTGT | 20 |
| 7 | F5 (Actin) | Biotin-AAgAgYCTATTgAgCTA | 17 |
| 8 | R1(Actin) | Biotin-CACYggATggCCAATCCAA | 19 |

**Table 18 (probes)**

| N° | NAME | Sequence(s) 5' → 3' | mer |
|---|---|---|---|
| 1 | CPA | GAATTAAACCACATGCTCCACTGCTTGTGC | 30 |
| 2 | MPA | AATCCCGACTCGTTAAAGCAGGATAA | 26 |
| 3 | LPP1 | AACAAGTTTCAGAAAGATTTGATGGCAAAG | 30 |
| 4 | actin | GAGCCGGAAATCGTGCGCGACATCAAGGAG | 30 |

| | | | |
|---|---|---|---|
| (Wherein : Y=C,T; R=A, G; H=A,T,C; M=A,C ; K=G;T) | | | |

**Table 19 (amplified sequences)**

| Target | Primer | Sequence(s) 5' → 3' |
|---|---|---|
| C.pneumoniae NCBI Reference sequence: gi8163533 | CPF | |
| | CPR | |
| | Length:195pb | |
| M.pneumoniae NCBI Reference sequence:gi150138 | MPF | |
| | MPR | |
| | Length:323 pb | |
| L.pneumophila NCBI Reference sequence: gi6958233 | LPmipAf | |
| | LpmipAr | |
| | Length:144 pb | |
| Actin313 NCBI Reference sequence: gi5016088 | F5 | |
| | R1 | |
| | Length: 313 pb | |

The performances of the analytical method were evaluated experimentally on clinical samples (bronchial aspirates, pharyngeal swabs, blood) that were classified positive by other reference tests. The extracted nucleic acid was first amplified and subsequently detected by hybridization on a chip (BCS RB CHIP). On the basis of the results interpreting scheme, the analytical kit provided the performances shown in Table 20, set out below:

**Table 20**

| Samples (Pharyngeal swabs, bronchial aspirates, blood) | Number of samples (negative + positive) | Diagnostic Specificity | Diagnostic Sensitivity | Analytical Sensitivity Copies/PCR |
|---|---|---|---|---|
| Chlamydia pneumoniae | 32 | 98.2% | 96.3% | 10³ |
| Mycoplasma pneumoniae | 20 | 98.4 % | 92.6 % | 10³ |
| Legionella pneumophilae | 25 | 99.1 % | 95.7 % | 10² |

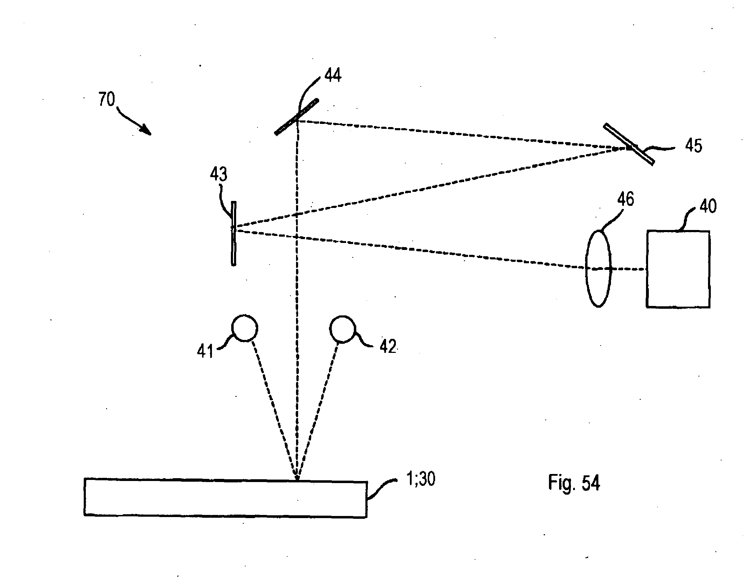

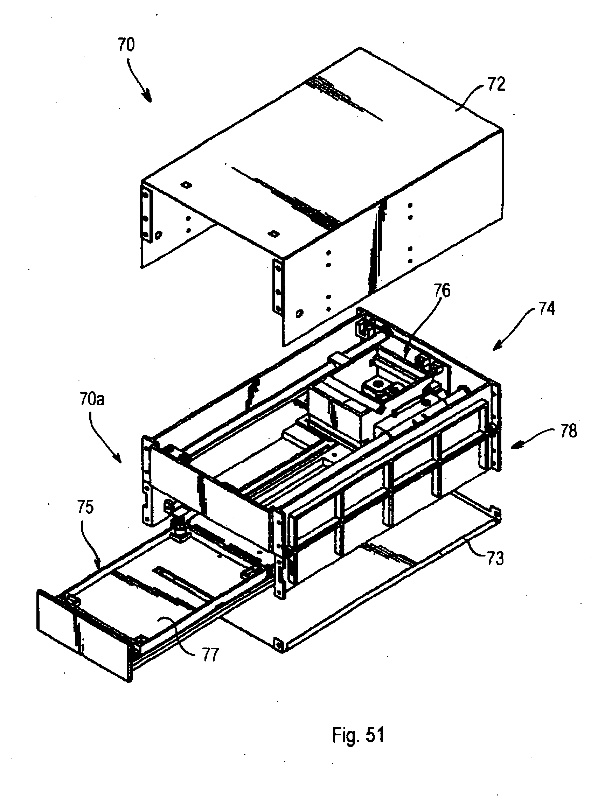

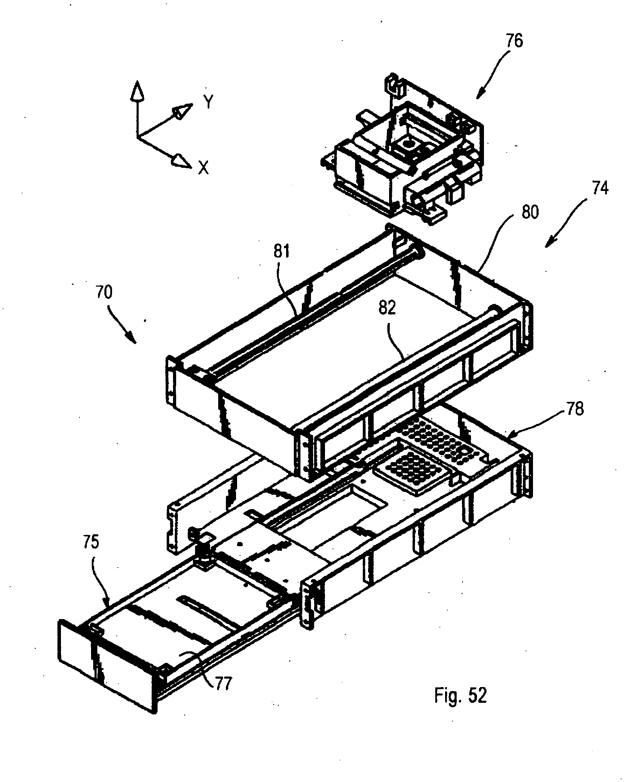

In order to enable reading of the chips 32' having a < 1 cm² area and/or to automate reading of the chips 32 having a > 1 cm² area, an apparatus is provided for acquiring an image from the chips 32 that are present in the container 1 (poly-chip) and/or from the chips 32' of the microplate 30. With reference to Figures 51-54, the apparatus comprises an optical unit 70, in which (Figure 51) a pair of light sources 41, 42 emitting white light, a plurality of mirrors 43-45, a focusing lens 46 and a sensor, for example a linear CCD 40, are present. The pair of light sources 41, 42 uniformly irradiate light inside the wells of the container 1 (poly-chip), or inside the wells 31 of the microplate 30, depending on the type of support used for the analysis. The lenses 43-45, suitably orientated inside the apparatus 70, retransmit the light reflected by the chips to the focusing lens 46 and then to the CCD 40. Since the aforementioned pair of light sources 41, 42 ensure uniform distribution of the light inside the wells, the contrast of the image and the intensity of the signal obtained from the reflection of the light are of good quality.

In addition to the optical unit 70, the apparatus comprises cooling means and a supply unit that are not shown.

With particular reference to Figures 51 to 53, the optical unit 70 comprises a base 73 and a lid 72, defining a space that is suitable for containing a scanning module 74. Onto a frontal portion 70a of the optical unit 70 an extractable drawer 75 is fitted in which a movable plate 77 is housed. In use, the microplate 30, or the poly-chip container 1 is located on the plate 77 when the latter is in an open position, i.e. extracted; thus by using a specific control an operator can automatically close the drawer 75 and start reading of the the chips 32. The scanning module 74 comprises an upper submodule 80 and a lower submodule 78.

In the upper submodule 80 an optic module 76 is housed, which can slide backwards and forwards, along a Y axis, owing to a pair of side guides 81, 82, fixed to opposite ends of the upper submodule 80.

The lower submodule 78 ensures suitable movement of the CCD of the optic module 76 along the Y axis and a suitable seat for the microplate 30 and/or the container 1. The lower submodule 78 comprises the extractable drawer 75, on a base portion 79 of which the movable plate 77 is fitted. The latter is movable along the Y axis, since it passively follows the movement of the drawer 75 when the latter is open and/or closed.

The optic module 76 comprises a control circuit (not shown) assembled on the rear part of the unit. The light sources 41, 42 are positioned in a lower portion, which is not shown, of the optic module. In use, the sources 41, 42, emit white light and irradiate the support that is to be read. A series of images of the chips 32, or 32', is generated by reflection of the light projected by the light sources 41, 42 through the mirrors 43-45. The image is then focused by the lens 46 and is received by the linear CCD 40.

With reference to Figure 55, CCD 40 starts scanning the wells (in the case shown, the wells 31 of a microplate 30) in direction A1→D1 along the Y axis. Subsequently, CCD 40 moves along a further X axis, orthogonal to the Y axis, and the wells in the direction A2→D2 are thus read, always along the Y axis This sequence of operations is repeated until the wells are read in the direction A12→B12. The scan of the wells in the direction E12→H12 is thus performed, proceding with the wells in the direction E11→H11 and continuing until the wells in the direction E1→H are scanned.

The scan of the microplate is performed twice, and consequently the CCD 40 moves backwards and forwards for a sufficient time so that the entire microplate 30 can be read, that is for about three minutes. Owing to this double scanning, it is possible to reduce the dimension of the images to be analysed.

The images of the chips 32, 32' obtained by the apparatus disclosed above can be acquired and managed by a suitable computer programme. The programme can perform automatically a comparison between the image of a given pattern, which has been produced on the chip 32, 32' by means of hybridization highlighted by a colorimetric reaction, with a series of images of reference patterns (templates) that have been previously defined and stored. The programme is able to create image files in a sequential manner. Once the image of a pattern has been acquired, the position of the dark areas, or spots, forming each pattern is compared with the position of the spots forming the patterns of the reference images. The operator can create a database of images to be used during subsequent analyses.

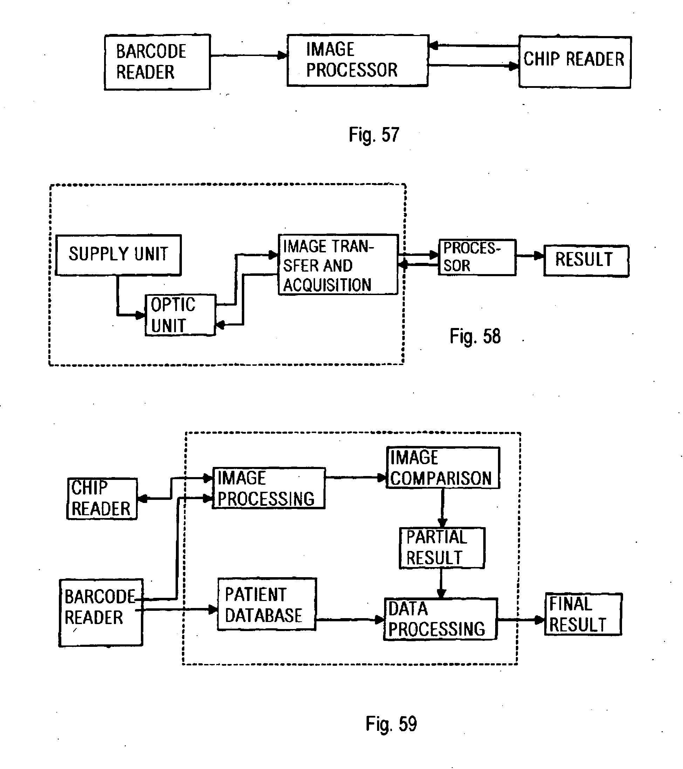

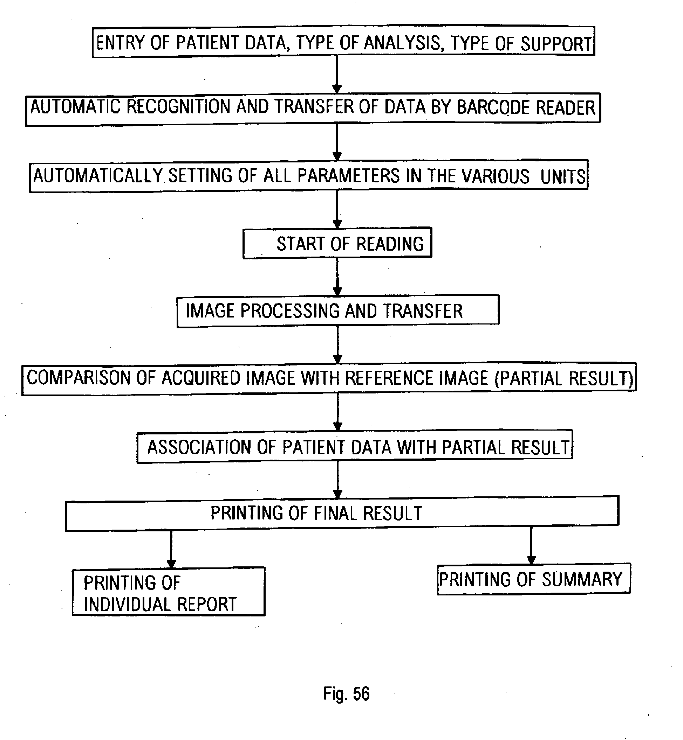

With reference to Figures 56 to 59, the possibility is provided of supplementing the reading apparatus disclosed above with further optic means, for example a bar code optic reader, of the known type. An arrangement (Figure 57) is obtained in this way comprising the bar code optic reader, the apparatus or chip reader disclosed above and an image processor, that is the computer on which the aforementioned programme for acquiring and managing images is run. In use, the three apparatuses reciprocally cooperate enabling the automatic management of data that are not directly analytical but nevertheless relate to the sample subjected to analysis to be associated with the interpretation of the images coming from the chips 32, 32'.

The operator, before starting to read the chips 32, 32', acquires information regarding the analysed sample, for example information on the origin of the sample and/or on the type of analysis made and therefore on the type of chip used, which information is contained in a bar code printed on an adhesive label. The latter can be applied, for example, to the paper documentation that normally accompanies each sample in a laboratory, or can be applied to the container of the sample. The programme for acquiring and managing images that is used to check the operation of the chip reader is also able to check the operation of the bar code optic reader. In this way, the data that are acquired through the latter are sent to the computer and stored therein.

Then the operator, by acting on the computer, sets the suitable reading parameters of the chip reader and, after positioning the microplate 30 or the container 1 in the drawer 75, starts reading. The chip reader acquires the images coming from the chips 32, 32' and sends them to the computer, where the comparison between the acquired images of the patterns and the reference images is made.

In this phase, the programme produces a so-called partial result, i.e. an analytical result, which is positive or negative depending on circumstances, which is not yet associated with the information on the sample that was previously acquired through the reading of the barcode. In a subsequent phase, the programme calls up from the computer memory the information on the sample and associates the information with the partial result, producing a final result in which the outcome of the analysis is associated with the information on the sample. This final result can be printed on paper in such a way as to produce an analytical report, and the programme can both print a single final result and a summarising printout, for example of the final results obtained during a certain period of laboratory activity.

Owing to the procedure disclosed above, the activity performed in a laboratory is significantly facilitated inasmuch as the time that is normally required for processing the analytical reports is substantially reduced.

## Claims

1. A method for the detection of HPV virus in a biological sample by Multiplex PCR comprising the following step:
- extracting genomic nucleic acids from said sample,
- simultaneously amplifying a plurality of genic sequences with the PCR primer mix
a) 5' CAGGGACAIAAIAATGGYATWTG-3',
b) 5' GAAAAATAAACTGTAAATCATATT-3',
c) 5'-GAGCTGTCGCTTAATTGCTC-3',
d) 5'-IWACIGTGGAAGAAGARAC-3',
e) 5'-AGAYRTTATAITTATTCIGTRTATGG-3'
wherein said primers are marked with a molecule selected from the group consisting of biotin, digoxigenin, fluorescein, so as to produce amplified genic sequences,
- obtaining hybridization between said amplified genic sequences and specific probes bound in preset sites of miniaturized solid support means
- highlighting said hybridization by means of a colorimetric reaction.

2. The method according to claim 1 wherein said probes comprise all of the following nucleic acid sequences:
○ PANHPVL1 probe: 5'-TTTGTWACTGTIGTRGAYACIACMCGIAGTAC-3',
○ PAN HPV E6/E7 probe: 5' -TGYCAIAARCCITTRTGIMIAGIRGAAAA-3'
○ HPV HR E6/E7 probes:
• 5'-CGTACAGAAACCCAGGTGTAATCATGCRTG-3',
• 5'-CAGTATAATCATGCAYGGTAAAGTACCAAC-3' ,
• 5' -GTGTAATAAMGCCATGCGTGGTAATGTACCACA-3',
• 5'-ACGIAGIGAAACACAIGTITATTTTTTTATGCATGGACC-3',
and wherein HPV subtypes specific probes are selected from the group consisting of the following sequences:
• 5'-ATCCGTAACTACATCTTCCACATACACCAA-3' ,
• 5'-ATCTGTGTCTAAATCTGCTACATACACTAA-3' ,
• 5'-GTCATTATGTGCTGCCATATCTACTTCAGA-3',
• 5'-TGCTTCTACACAGTCTCCTGTACCTGGGCA-3' ,
• 5'-TAGTACATTATCTGCAGCATCTGCATCC-3,
• 5'-TATCTGCAACAACACAAACGTTATCCAC-3'
• 5'-TGTTTGTGCTGCAATTGCAAACAGTGATAC-3'
• 5'-TTTATGCACACAAGTAACTAGTGACAGTAC-3' ,
• 5'-TACACAATCCACAAGTACAAATGCACCATA-3' ,
• 5' -GTCTGTGTGTTCTGCTGTGCTTTCTAGTGA-3' ,
• 5'-TCTACCTCTATAGAGTCTTCCATACCTTCT-3' ,
• 5'-GCTGCCACACAGTCCCCCACACCAACCCCA-3' ,
• 5'-CTGCAACATCTGGTGATACATATACAGCTG-3',
• 5' -TCTACTGACCCTACTGTGCCCAGTACATAT-3',
• 5' -GCCACTACACAGTCCCCTCCGTCTACATAT-3' ,
• 5'-ACACAAAATCCTGTGCCAAGTACATATGAC-3',
• 5'-AGCACTGCCACTGCTGCGGTTTCCCCAACA-3',
• 5'-TGCTGAGGTTAAAAAGGAAAGCACATATAA-3' ,
• 5'-ACTCTTTCCGCAACCACACAGTCTATGTCT-3',
• 5'-TACAGCATCCACGCAGGATAGCTTTAATAA-3' ,
• 5'-GTACTGCTACAGAACAGTTAAGTAAATATG-3',
• 5'-ATTATGCACTGAAGTAACTAAGGAAGGTAC-3' ,
• 5'-TCTGTGTGTGCTTCTACTACTTCTTCTAT-3',
• 5'-ACTATTATTGCAGCTAAAAGCACATTAACT-3',
• 5'-ACTTTATGTTCTGAGGAAAAATCAGAGGCT-3' ,
• 5'-TTTGTCTACTACTACTGAATCAGCTGT-3'
• 5'-AGTACTGTATCTGCACAATCTGCATCTGCC-3'
• 5'-ATTGTCTGCCTGCACCGAAACGGCCAT-3'
• 5'-AGGCTAGTAGCTCTACTACAACGTATGC-3'
• 5'-GTTTACTCCATCTGTTGCACAAACATTTAC-3',
• 5'-CAATGTATCATGCCTCCTTGCACCATTCTA-3', said probes bound in preset sites of miniaturized solid support means.

3. The method according to claim 2 further comprising sequences as an amplification control.

4. The method according to claim 3, wherein said amplification internal control are β-globin specific primers with the following sequence:
f) 5'-CTTTCAGGGCAATAATGA-3',
g) 5'-TGGTAGCTGGATTGTAGC-3'
and probe with the following sequence
h) 5'GGGTTACGTAAGCTACCTAGCTACTGCATG -3'

5. The method according to claims 1-4 further comprising the steps of:
- scanning , through optic reader means, of the surface of miniaturized solid support means, said surface comprising sites at which hybridization between amplified genic sequence and specific probes has been induced, so as to obtain an image of said sites;
- comparing said image with a reference image, so as to verify whether and in which of said sites said hybridization has been obtained
- interpreting an analytical result as a result of said comparison.

6. The method according to claim 5 wherein said analytical result is expressed in terms of the presence or absence of a HPV pathogenic agent.

7. The method of claims 5-6 wherein the steps of scanning and comparing is computer-implemented.

8. A kit comprising a HPV PCR primer mix as defined in claim 1, further comprising as internal control the following primers:
5'-CTTTCAGGGCAATAATGA-3',
5'-TGGTAGCTGGATTGTAGC-3'
for carrying out the method according to claim 3.

9. The kit according to claim 8 further comprising the HPV subtypes specific probes as defined in claim 2.

## Patentansprüche

1. Verfahren zur Detektion von HPV-Virus in einer biologischen Probe durch Multiplex-PCR, welches die nachfolgenden Schritte umfasst:
- Extrahieren von genomischen Nukleinsäuren aus der Probe,
- gleichzeitig Amplifizieren einer Vielzahl von Gensequenzen mit einer PCR-Primermischung
a) 5' CAGGGACAIAAIAATGGYATWTG-3',
b) 5' GAAAAATAAACTGTAAATCATATT-3',
c) 5'-GAGCTGTCGCTTAATTGCTC-3',
d) 5'-IWACIGTGGAAGAAGARAC-3',
e) 5'-AGAYRTTATAITTATTCIGTRTATGG-3' wobei die Primer mit einem Molekül markiert sind, welches aus der Gruppe ausgewählt wird, welche aus Biotin, Digoxigenin, Fluorescein besteht, um so amplifizierte Gensequenzen herzustellen,
- Bewirken einer Hybridisierung zwischen den amplifizierten Gensequenzen und spezifischen Sonden, welche an vorbestimmten Stellen von miniaturisiertem festem Trägermittel gebunden sind,
- Visualisieren der Hybridisierung mittels einer kolorimetrischen Reaktion.

2. Verfahren nach Anspruch 1, wobei die Sonden alle der nachfolgenden Nukleinsäuresequenzen umfassen:
○ PAN HPVL1-Sonde:
5'-TTTGTWACTGTIGTRGAYACIACMCGIAGTAC-3',
○ PAN HPV E6/E7-Sonde:
5' -TGYCAIAARCCITTRTGIMIAGIRGAAAA-3',
**○** HPV HR E6/E7-Sonden:
• 5'-CGTACAGAAACCCAGGTGTAATCATGCRTG-3',
• 5'-CAGTATAATCATGCAYGGTAAAGTACCAAC-3',
• 5'-GTGTAATAAMGCCATGCGTGGTAATGTACCACA-3',
• 5'-ACGIAGIGAAACACAIGTITATTTTTTTATGCATGGACC-3',
und wobei für HPV-Subtypen spezifische Sonden aus der Gruppe ausgewählt werden, welche aus den nachfolgenden Sequenzen besteht:
• 5'-ATCCGTAACTACATCTTCCACATACACCAA-3',
• 5'-ATCTGTGTCTAAATCTGCTACATACACTAA-3',
• 5'-GTCATTATGTGCTGCCATATCTACTTCAGA-3',
• 5'-TGCTTCTACACAGTCTCCTGTACCTGGGCA-3',
• 5'-TAGTACATTATCTGCAGCATCTGCATCC-3,
• 5'-TATCTGCAACAACACAAACGTTATCCAC-3',
• 5'-TGTTTGTGCTGCAATTGCAAACAGTGATAC-3',
• 5'-TTTATGCACACAAGTAACTAGTGACAGTAC-3',
• 5'-TACACAATCCACAAGTACAAATGCACCATA-3',
• 5'-GTCTGTGTGTTCTGCTGTGCTTTCTAGTGA-3',
• 5'-TCTACCTCTATAGAGTCTTCCATACCTTCT-3',
• 5'-GCTGCCACACAGTCCCCCACACCAACCCCA-3',
• 5'-CTGCAACATCTGGTGATACATATACAGCTG-3',
• 5'-TCTACTGACCCTACTGTGCCCAGTACATAT-3',
• 5'-GCCACTACACAGTCCCCTCCGTCTACATAT-3',
• 5'-ACACAAAATCCTGTGCCAAGTACATATGAC-3',
• 5'-AGCACTGCCACTGCTGCGGTTTCCCCAACA-3',
• 5'-TGCTGAGGTTAAAAAGGAAAGCACATATAA-3',
• 5'-ACTCTTTCCGCAACCACACAGTCTATGTCT-3',
• 5'-TACAGCATCCACGCAGGATAGCTTTAATAA-3',
• 5'-GTACTGCTACAGAACAGTTAAGTAAATATG-3',
• 5'-ATTATGCACTGAAGTAACTAAGGAAGGTAC-3',
• 5'-TCTGTGTGTGCTTCTACTACTTCTTCTAT-3',
• 5'-ACTATTATTGCAGCTAAAAGCACATTAACT-3',
• 5'-ACTTTATGTTCTGAGGAAAAATCAGAGGCT-3',
• 5'-TTTGTCTACTACTACTGAATCAGCTGT-3',
• 5'-AGTACTGTATCTGCACAATCTGCATCTGCC-3',
• 5'-ATTGTCTGCCTGCACCGAAACGGCCAT-3',
• 5'-AGGCTAGTAGCTCTACTACAACGTATGC-3',
• 5'-GTTTACTCCATCTGTTGCACAAACATTTAC-3',
• 5'-CAATGTATCATGCCTCCTTGCACCATTCTA-3', wobei die Sonden an vorbestimmten Stellen des miniaturisierten festen Trägermittels gebunden sind.

3. Verfahren nach Anspruch 2, des Weiteren umfassend Sequenzen als eine Amplifikationskontrolle.

4. Verfahren nach Anspruch 3, wobei die interne Amplifikationskontrolle für β-Globin spezifische Primer mit den nachfolgenden Sequenzen sind:
f) 5'-CTTTCAGGGCAATAATGA-3',
g) 5'-TGGTAGCTGGATTGTAGC-3',
und eine Sonde mit der nachfolgenden Sequenz
h) 5'-GGGTTACGTAAGCTACCTAGCTACTGCATG-3'.

5. Verfahren nach einem der Ansprüche 1 bis 4, welches des Weiteren die folgenden Schritte umfasst:
- Abtasten, durch optische Lesemittel, der Oberfläche des miniaturisierten festen Trägermittels, wobei die Oberfläche Stellen umfasst, an denen eine Hybridisierung zwischen amplifizierten Gensequenzen und spezifischen Sonden induziert worden ist, um so ein Bild der Stellen zu erhalten,
- Vergleichen des Bildes mit einem Referenzbild, um so zu verifizieren, ob und an welcher der Stellen die Hybridisierung erhalten worden ist,
- Interpretieren eines analytischen Ergebnisses als ein Ergebnis des Vergleiches.

6. Verfahren nach Anspruch 5, wobei das analytische Ergebnis in Form von Anwesenheit oder Abwesenheit eines HPV-pathogenen Mittels ausgedrückt wird.

7. Verfahren nach Anspruch 5 oder 6, wobei die Schritte des Abtastens und Vergleichens computerimplementiert sind.

8. Kit enthaltend eine HPV-PCR-Primermischung nach Anspruch 1, welches als interne Kontrolle die nachfolgenden Primer umfasst:
5'-CTTTCAGGGCAATAATGA-3',
5'-TGGTAGCTGGATTGTAGC-3'
zum Durchführen eines Verfahrens nach Anspruch 3.

9. Kit nach Anspruch 8, welches des Weiteren für HPV-Subtypen spezifische Sonden, wie in dem Anspruch 2 definiert, umfasst.

## Revendications

1. Procédé de détection du virus HPV dans un échantillon biologique par PCR Multiplex, comprenant l'étape suivante:
- extraire des acides nucléiques génomiques dudit échantillon,
- amplifier simultanément une pluralité de séquences géniques avec le mélange d'amorces PCR
a) 5' CAGGGACAIAAIAATGGYATWTG-3',
b) 5' GAAAAATAAACTGTAAATCATATT-3',
c) 5'-GAGCTGTCGCTTAATTGCTC-3',
d) 5'-IWACIGTGGAAGAAGARAC-3',
e) 5'-AGAYRTTATAITTATTCIGTRTATGG-3'
où lesdites amorces sont marquées avec une molécule sélectionnée dans le groupe consistant en biotine, digoxigénine, fluorescéine, de manière à produire des séquences géniques amplifiées,
- obtenir une hybridation entre lesdites séquences géniques amplifiées et des ondes spécifiques liées dans des sites préétablis d'un moyen de support solide miniaturisé
- mettre en évidence ladite hybridation au moyen d'une réaction colorimétrique.

2. Procédé selon la revendication 1, dans lequel lesdites sondes comprennent toutes les séquences d'acides nucléiques suivantes:
○ PAN HPVL1 sonde: 5' -TTTGTWACTGTIGTRGAYACIACMCGIAGTAC-3',
○ PAN HPV E6/E7 sonde: 5' -TGYCAIAARCCITTRTGIMIAGIRGAAAA-3'
○ HPV HR E6/E7 sondes:
• 5'-CGTACAGAAACCCAGGTGTAATCATGCRTG-3',
• 5'-CAGTATAATCATGCAYGGTAAAGTACCAAC-3',
• 5'-GTGTAATAAMGCCATGCGTGGTAATGTACCACA-3',
• 5'-ACGIAGIGAAACACAIGTITATTTTTTTATGCATGGACC-3',
et où des sondes spécifiques de sous-types de HPV sont sélectionnées dans le groupe consistant en séquences suivantes:
• 5'-ATCCGTAACTACATCTTCCACATACACCAA-3',
• 5'-ATCTGTGTCTAAATCTGCTACATACACTAA-3',
• 5'-GTCATTATGTGCTGCCATATCTACTTCAGA-3',
• 5'-TGCTTCTACACAGTCTCCTGTACCTGGGCA-3' ,
• 5'-TAGTACATTATCTGCAGCATCTGCATCC-3',
• 5'-TATCTGCAACAACACAAACGTTATCCAC-3',
• 5'-TGTTTGTGCTGCAATTGCAAACAGTGATAC-3',
• 5'-TTTATGCACACAAGTAACTAGTGACAGTAC-3',
• 5'-TACACAATCCACAAGTACAAATGCACCATA-3',
• 5'-GTCTGTGTGTTCTGCTGTGCTTTCTAGTGA-3',
• 5'-TCTACCT CTATAGAGTCTTCCATACCTTCT-3',
• 5'-GCTGCCACACAGTCCCCCACACCAACCCCA-3',
• 5'-CTGCAACATCTGGTGATACATATACAGCTG-3',
• 5'-TCTACTGACCCTACTGTGCCCAGTACATAT-3',
• 5'-GCCACTACACAGTCCCCTCCGTCTACATAT-3',
• 5'-ACACAAAATCCT GTGCCAAGTACATATGAC-3',
• 5'-AGCAC TGCCACT GC TGC GGT TTCCCCAACA-3',
• 5'-TGCTGAGGTTAAAAAGGAAAGCACATATAA-3',
• 5'-ACTCTTTCCGCAACCACACAGTCTATGTCT-3 ',
• 5'-TACAGCATCCACGCAGGATAGCTTTAATAA-3',
• 5'-GTACTGCTACAGAACAGTTAAGTAAATATG-3',
• 5'-ATTATGCACTGAAGTAACTAAGGAAGGTAC-3',
• 5'-TCTGTGTGTGCTTCTACTACTTCTTCTAT-3',
• 5'-ACTATTATTGCAGCTAAAAGCACATTAACT-3',
• 5'-ACTTTATGTTCTGAGGAAAAATCAGAGGCT-3 ',
• 5'-TTTGTCTACTACTACTGAATCAGCTGT-3',
• 5'-AGTACTGTATCTGCACAATCTGCATCTGCC-3',
• 5'-ATTGTCTGCCTGCACCGAAACGGCCAT-3',
• 5'-AGGCTAGTAGCTCTACTACAACGTATGC-3',
• 5'-GTTTACTCCATCTGTTGCACAAACATTTAC-3',
• 5'-CAATGTATCATGCCTCCTTGCACCATTCTA-3',
lesdites sondes étant liées dans des sites préétablis d'un moyen de support solide miniaturisé.

3. Procédé selon la revendication 2, comprenant en outre des séquences comme contrôle d'amplification.

4. Procédé selon la revendication 3, dans lequel lesdits contrôles internes d'amplification sont des amorces spécifiques de la β-globine avec la séquence suivante:
f) 5'-CTTTCAGGGCAATAATGA-3',
g) 5'-TGGTAGCTGGATTGTAGC-3'
et la sonde avec la séquence suivante
h) 5' GGGTTACGTAAGCTACCTAGCTACTGCATG-3'

5. Procédé selon les revendications 1 à 4, comprenant en outre les étapes de:
- balayer, par un moyen de lecture optique, la surface du moyen de support solide miniaturisé, ladite surface comprenant des sites où une hybridation entre la séquence génique amplifiée et des sondes spécifiques a été induite afin d'obtenir une image desdits sites;
- comparer ladite image avec une image de référence de manière à vérifier si et dans quel site ladite hybridation a été obtenue,
- interpréter un résultat analytique comme un résultat de ladite comparaison.

6. Procédé selon la revendication 5, dans lequel ledit résultat analytique est exprimé en termes de présence ou d'absence d'un agent pathogène de HPV.

7. Procédé selon les revendications 5-6, dans lequel les étapes de balayage et de comparaison sont exécutées par ordinateur.

8. Kit comprenant un mélange d'amorces de HPV PCR tel que défini dans la revendication 1, comprenant en outre comme contrôle interne les amorces suivants:
5' -CTTTCAGGGCAATAATGA-3',
5' -TGGTAGCTGGATTGTAGC-3',
pour exécuter le procédé selon la revendication 3.

9. Kit selon la revendication 8, comprenant en outre les sondes spécifiques à des sous-types de HPV telles que définies dans la revendication 2.


### SEQUENCE LISTING

<110> bcs BIOTECH S.p.A.
<120> System for searching for and identifying pathogenic agents
<130> 1061460 BE
<140>
   <141>
<150> IT M02004A000126
   <151> 2004-05-21
<160> 108
<170> PatentIn version 3.3
<210> 1
   <211> 23
   <212> DNA
   <213> Artificial Sequence
<220>
   <223> Primer for identifying L1 region of Human papillomavirus genome
<220>
   <221> modified_base
   <222> (9)..(9)
   <223> I
<220>
   <221> modified_base
   <222> (12)..(12)
   <223> I
<400> 1
   cagggacaaa aaaatggyat wtg 23
<210> 2
   <211> 24
   <212> DNA
   <213> Artificial Sequence
<220>
   <223> Primer for identifying L1 region of Human papillomavirus genome
<400> 2
   gaaaaataaa ctgtaaatca tatt 24
<210> 3
   <211> 20
   <212> DNA
   <213> Artificial Sequence
<220>
   <223> Primer for identifying a consensus region of E6/E7 region of Human papillomavirus genome
<400> 3
   gagctgtcgc ttaattcgtc 20
<210> 4
   <211> 18
   <212> DNA
   <213> Artificial Sequence
<220>
   <223> Primer for identifying a consensus region of E6/E7 region of Human papillomavirus genome
<220>
   <221> modified_base
   <222> (1)..(1)
   <223> I
<220>
   <221> modified_base
   <222> (5)..(5)
   <223> I
<400> 4
   awacagtgga agaagara 18
<210> 5
   <211> 26
   <212> DNA
   <213> Artificial Sequence
<220>
   <223> Primer for identifying a consensus region of E6/E7 region of Human papillomavirus genome
<220>
   <221> modified_base
   <222> (11)..(11)
   <223> I
<220>
   <221> modified_base
   <222> (18)..(18)
   <223> I
<400> 5
   agayrttata attattcagt rtatgg 26
<210> 6
   <211> 18
   <212> DNA
   <213> Artificial Sequence
<220>
   <223> Primer specific for a fragment of human Beta-globin gene
<400> 6
   ctttcagggc aataatga 18
<210> 7
   <211> 18
   <212> DNA
   <213> Artificial Sequence
<220>
   <223> Primer specific for a fragment of human Beta-globin gene
<400> 7
   tggtagctgg attgtagc 18
<210> 8
   <211> 30
   <212> DNA
   <213> Artificial Sequence
<220>
   <223> probe specific for Human papillomavirus type 6 genome
<400> 8
   atccgtaact acatcttcca catacaccaa 30
<210> 9
   <211> 30
   <212> DNA
   <213> Artificial Sequence
<220>
   <223> probe specific for Human papillomavirus type 11 genome
<400> 9
   atctgtgtct aaatctgcta catacactaa 30
<210> 10
   <211> 30
   <212> DNA
   <213> Artificial sequence
<220>
   <223> probe specific for Human papillomavirus type 16 genome
<400> 10
   gtcattatgt gctgccatat ctacttcaga 30
<210> 11
   <211> 30
   <212> DNA
   <213> Artificial Sequence
<220>
   <223> probe specific for Human papillomavirus type 18 genome
<400> 11
   tgcttctaca cagtctcctg tacctgggca 30
<210> 12
   <211> 28
   <212> DNA
   <213> Artificial Sequence
<220>
   <223> probe specific for Human papillomavirus type 26 genome
<400> 12
   tagtacatta tctgcagcat ctgcatcc 28
<210> 13
   <211> 28
   <212> DNA
   <213> Artificial Sequence
<220>
   <223> probe specific for Human papillomavirus type 30 genome
<400> 13
   tatctgcaac cacacaaacg ttatccac 28
<210> 14
   <211> 30
   <212> DNA
   <213> Artificial Sequence
<220>
   <223> probe specific for Human papillomavirus type 31 genome
<400> 14
   tgtttgtgct gcaattgcaa acagtgatac 30
<210> 15
   <211> 30
   <212> DNA
   <213> Artificial Sequence
<220>
   <223> probe specific for Human papillomavirus type 33 genome
<400> 15
   tttatgcaca caagtaacta gtgacagtac 30
<210> 16
   <211> 30
   <212> DNA
   <213> Artificial Sequence
<220>
   <223> probe specific for Human papillomavirus type 34 genome
<400> 16
   tacacaatcc acaagtacaa atgcaccata 30
<210> 17
   <211> 30
   <212> DNA
   <213> Artificial Sequence
<220>
   <223> probe specific for Human papillomavirus type 35 genome
<400> 17
   gtctgtgtgt tctgctgtgt cttctagtga 30
<210> 18
   <211> 30
   <212> DNA
   <213> Artificial Sequence
<220>
   <223> probe specific for Human papillomavirus type 39 genome
<400> 18
   tctacctcta tagagtcttc cataccttct 30
<210> 19
   <211> 30
   <212> DNA
   <213> Artificial Sequence
<220>
   <223> probe specific for Human papillomavirus type 40 genome
<400> 19
   gctgccacac agtcccccac accaacccca 30
<210> 20
   <211> 30
   <212> DNA
   <213> Artificial Sequence
<220>
   <223> probe specific for Human papillomavirus type 42 genome
<400> 20
   ctgcaacatc tggtgataca tatacagctg 30
<210> 21
   <211> 30
   <212> DNA
   <213> Artificial Sequence
<220>
   <223> probe specific for Human papillomavirus type 43 genome
<400> 21
   tctactgacc ctactgtgcc cagtacatat 30
<210> 22
   <211> 30
   <212> DNA
   <213> Artificial Sequence
<220>
   <223> probe specific for Human papillomavirus type 44 genome
<400> 22
   gccactacac agtcccctcc gtctacatat 30
<210> 23
   <211> 30
   <212> DNA
   <213> Artificial Sequence
<220>
   <223> probe specific for Human papillomavirus type 45 genome
<400> 23
   acacaaaatc ctgtgccaag tacatatgac 30
<210> 24
   <211> 30
   <212> DNA
   <213> Artificial Sequence
<220>
   <223> probe specific for Human papillomavirus type 51 genome
<400> 24
   agcactgcca ctgctgcggt ttccccaaca 30
<210> 25
   <211> 30
   <212> DNA
   <213> Artificial Sequence
<220>
   <223> probe specific for Human papillomavirus type 52 genome
<400> 25
   tgctgaggtt aaaaaggaaa gcacatataa 30
<210> 26
   <211> 30
   <212> DNA
   <213> Artificial Sequence
<220>
   <223> probe specific for Human papillomavirus type 53 genome
<400> 26
   actctttccg caaccacaca gtctatgtct 30
<210> 27
   <211> 30
   <212> DNA
   <213> Artificial Sequence
<220>
   <223> probe specific for Human papillomavirus type 54 genome
<400> 27
   tacagcatcc acgcaggata gctttaataa 30
<210> 28
   <211> 30
   <212> DNA
   <213> Artificial Sequence
<220>
   <223> probe specific for Human papillomavirus type 56 genome
<400> 28
   gtactgctac agaacagtta agtaaatatg 30
<210> 29
   <211> 30
   <212> DNA
   <213> Artificial Sequence
<220>
   <223> probe specific for Human papillomavirus type 58 genome
<400> 29
   attatgcact gaagtaacta aggaaggtac 30
<210> 30
   <211> 29
   <212> DNA
   <213> Artificial Sequence
<220>
   <223> probe specific for Human papillomavirus type 59 genome
<400> 30
   tctgtgtgtg cttctactac ttcttctat 29
<210> 31
   <211> 30
   <212> DNA
   <213> Artificial Sequence
<220>
   <223> probe specific for Human papillomavirus type 66 genome
<400> 31
   actattaatg cagctaaaag cacattaact 30
<210> 32
   <211> 30
   <212> DNA
   <213> Artificial Sequence
<220>
   <223> probe specific for Human papillomavirus type 67 genome
<400> 32
   actttatgtt ctgaggaaaa atcagaggct 30
<210> 33
   <211> 27
   <212> DNA
   <213> Artificial Sequence
<220>
   <223> probe specific for Human papillomavirus type 68 genome
<400> 33
   tttgtctact actactgaat cagctgt 27
<210> 34
   <211> 30
   <212> DNA
   <213> Artificial Sequence
<220>
   <223> probe specific for Human papillomavirus type 69 genome
<400> 34
   agtactgtat ctgcacaatc tgcatctgcc 30
<210> 35
   <211> 27
   <212> DNA
   <213> Artificial Sequence
<220>
   <223> probe specific for Human papillomavirus type 70 genome
<400> 35
   attgtctgcc tgcaccgaaa cggccat 27
<210> 36
   <211> 28
   <212> DNA
   <213> Artificial Sequence
<220>
   <223> probe specific for Human papillomavirus type 73 genome
<400> 36
   aggctagtag ctctactaca acgtatgc 28
<210> 37
   <211> 30
   <212> DNA
   <213> Artificial Sequence
<220>
   <223> probe specific for Human papillomavirus type 82 genome
<400> 37
   gtttactcca tctgttgcac aaacatttac 30
<210> 38
   <211> 30
   <212> DNA
   <213> Artificial Sequence
<220>
   <223> probe specific for human beta-globin gene
<400> 38
   caatgtatca tgcctctttg caccattcta 30
<210> 39
   <211> 30
   <212> DNA
   <213> Artificial Sequence
<220>
   <223> probe for internal control
<400> 39
   gggttacgta agctacctag ctactgcatg 30
<210> 40
   <211> 32
   <212> DNA
   <213> Artificial Sequence
<220>
   <223> probe specific for L1 region of Human papillomavirus genome
<220>
   <221> modified_base
   <222> (12)..(12)
   <223> I
<220>
   <221> modified_base
   <222> (21)..(21)
   <223> I
<220>
   <221> modified_base
   <222> (27)..(27)
   <223> I
<400> 40
   tttgtwactg tagtrgayac aacmcgaagt ac 32
<210> 41
   <211> 30
   <212> DNA
   <213> Artificial Sequence
<220>
   <223> probe specific for E6/E7 regions of Human papillomavirus genome
<400> 41
   cgtacagaaa cccaggtgtaa atcatgcrtg 30
<210> 42
   <211> 30
   <212> DNA
   <213> Artificial Sequence
<220>
   <223> probe specific for E6/E7 regions of Human papillomavirus genome
<400> 42
   cagtataatc atgcayggta aagtaccaac 30
<210> 43
   <211> 33
   <212> DNA
   <213> Artificial Sequence
<220>
   <223> probe specific for E6/E7 regions Human papillomavirus genome
<400> 43
   gtgtaataam gccatgcgtg gtaatgtacc aca 33
<210> 44
   <211> 39
   <212> DNA
   <213> Artificial Sequence
<220>
   <223> probe specific for E6/E7 regions of Human papillomavirus genome
<220>
   <221> modified_base
   <222> (4)..(4)
   <223> I
<220>
   <221> modified_base
   <222> (7)..(7)
   <223> I
<220>
   <221> modified_base
   <222> (16)..(16)
   <223> I
<220>
   <221> modified_base
   <222> (19)..(19)
   <223> I
<400> 44
   acgaagagaa acacaagtat atttttttat gcatggacc 39
<210> 45
   <211> 29
   <212> DNA
   <213> Artificial Sequence
<220>
   <223> probe specific for E6/E7 regions of Human papillomavirus genome
<220>
   <221> modified_base
   <222> (6)..(6)
   <223> I
<220>
   <221> modified_base
   <222> (12)..(12)
   <223> I
<220>
   <221> modified_base
   <222> (18)..(18)
   <223> I
<220>
   <221> modified_base
   <222> (20)..(20)
   <223> I
<220>
   <221> modified_base
   <222> (23)..(23)
   <223> I
<400> 45
   tgycaaaarc cattrtgama agargaaaa 29
<210> 46
   <211> 178
   <212> DNA
   <213> Homo sapiens
<400> 46
   ctttcagggc aataatgata caatgtatca tgcctctttg caccattcta aagaataaca 60
   gtgataattt ctgggttaag gcaatagcaa tatctctgca tataaatatt tctgcatata 120
   aattgtaact gatgtaagag gtttcatatt gctaatagca gctacaatcc agctacca 178
<210> 47
   <211> 173
   <212> DNA
   <213> Artificial Sequence
<220>
   <223> PCR internal control
<400> 47
   ctttcagggc aataatgata ggtacgtaag ctacctagct actgcatgaa taacagtgat 60
   aatttctggg ttaaggcaat agcaatatct ctgcatataa atatttctgc atataaattg 120
   taactgatgt aagaggtttc atattgctaa tagcagctac aatccagcta cca 173
<210> 48
   <211> 29
   <212> DNA
   <213> Artificial Sequence
<220>
   <223> probe
<400> 48
   atgaagcayg tcagggcrtg gatacctcg 29
<210> 49
   <211> 27
   <212> DNA
   <213> Artificial Sequence
<220>
   <223> probe
<400> 49
   tcctccggcc cctgaatgcg gctaatc 27
<210> 50
   <211> 33
   <212> DNA
   <213> Artificial Sequence
<220>
   <223> probe
<400> 50
   tgtcgtaacg sgcaastcyg yrgcggaacc gac 33
<210> 51
   <211> 28
   <212> DNA
   <213> Artificial Sequence
<220>
   <223> probe
<400> 51
   tactttgggt gtccgtgttt chttttat 28
<210> 52
   <211> 30
   <212> DNA
   <213> Artificial Sequence
<220>
   <223> probe
<400> 52
   cttataagca gactcaaccc ggtgctgatg 30
<210> 53
   <211> 28
   <212> DNA
   <213> Artificial Sequence
<220>
   <223> probe
<400> 53
   tggcattcca atatcacaat taacagtg 28

<210> 54
   <211> 29
   <212> DNA
   <213> Artificial sequence
<220>
   <223> probe
<400> 54
   rcarcacccg tacgtgctyg atgctggsa 29
<210> 55
   <211> 30
   <212> DNA
   <213> Artificial Sequence
<220>
   <223> probe
<400> 55
   ctcggcacta tcgcaggagg gaccgggaat 30
<210> 56
   <211> 30
   <212> DNA
   <213> Artificial Sequence
<220>
   <223> probe
<400> 56
   cctacgccac tacacagcct ggtcaggttg 30
<210> 57
   <211> 27
   <212> DNA
   <213> Artificial Sequence
<220>
   <223> probe
<400> 57
   taacatgacc aargactggt tcctggt 27
<210> 58
   <211> 25
   <212> DNA
   <213> Artificial sequence
<220>
   <223> probe
<400> 58
   acaayatygg mtaccagggc ttyta 25
<210> 59
   <211> 26
   <212> DNA
   <213> Artificial sequence
<220>
   <223> probe
<400> 59
   aacatgacca aggactggtt cctggt 26
<210> 60
   <211> 25
   <212> DNA
   <213> Artificial Sequence
<220>
   <223> probe
<400> 60
   acaatattgg ctaccagggc ttcta 25
<210> 61
   <211> 30
   <212> DNA
   <213> Artificial Sequence
<220>
   <223> probe
<400> 61
   gagccggaaa tcgtgcgcga catcaaggag 30
<210> 62
   <211> 16
   <212> DNA
   <213> Artificial Sequence
<220>
   <223> probe
<400> 62
   ctccagaatc atctcc 16
<210> 63
   <211> 22
   <212> DNA
   <213> Artificial Sequence
<220>
   <223> probe
<400> 63
   ctgccatctg gattcccatg ta 22
<210> 64
   <211> 21
   <212> DNA
   <213> Artificial Sequence
<220>
   <223> probe
<400> 64
   gtatggtatt gaatatacca c 21
<210> 65
   <211> 24
   <212> DNA
   <213> Artificial Sequence
<220>
   <223> probe
<400> 65
   gtcaccatca ttgattgagt actt 24
<210> 66
   <211> 25
   <212> DNA
   <213> Artificial Sequence
<220>
   <223> probe
<400> 66
   ttcatcatct gaaatctcat tttta 25
<210> 67
   <211> 25
   <212> DNA
   <213> Artificial Sequence
<220>
   <223> probe
<400> 67
   tgaattatca tttatttctg ttgct 25
<210> 68
   <211> 24
   <212> DNA
   <213> Artificial sequence
<220>
   <223> probe
<400> 68
   ttcgtgtcac taatttgagt tgga 24
<210> 69
   <211> 20
   <212> DNA
   <213> Artificial Sequence
<220>
   <223> probe
<400> 69
   taccaacgct gaacatcacg 20
<210> 70
   <211> 20
   <212> DNA
   <213> Artificial Sequence
<220>
   <223> primer
<220>
   <221> modified_base
   <222> (3)..(3)
   <223> I
<220>
   <221> modified_base
   <222> (18)..(18)
   <223> I
<400> 70
   ggatggtrst ggaarttacc 20
<210> 71
   <211> 20
   <212> DNA
   <213> Artificial Sequence
<220>
   <223> primer
<220>
   <221> modified_base
   <222> (18)..(18)
   <223> I
<400> 71
   arrtttatcc aytgrtgagg 20
<210> 72
   <211> 25
   <212> DNA
   <213> Artificial Sequence
<220>
   <223> primer
<400> 72
   ggaaatgtct caggtacttt ctttg 25
<210> 73
   <211> 26
   <212> DNA
   <213> Artificial Sequence
<220>
   <223> primer
<400> 73
   gttttgctcc tctttatcat gctatg 26
<210> 74
   <211> 21
   <212> DNA
   <213> Artificial sequence
<220>
   <223> primer
<400> 74
   cttgttggtt tgaggccata t 21
<210> 75
   <211> 19
   <212> DNA
   <213> Artificial Sequence
<220>
   <223> primer
<400> 75
   ataaaagttg gcatgaaca 19
<210> 76
   <211> 28
   <212> DNA
   <213> Artificial Sequence
<220>
   <223> primer
<400> 76
   ggctttaaaa gagagaattt ccgtctgg 28
<210> 77
   <211> 17
   <212> DNA
   <213> Artificial Sequence
<220>
   <223> primer
<400> 77
   gatcctgttg gccatcc 17
<210> 78
   <211> 19
   <212> DNA
   <213> Artificial Sequence
<220>
   <223> primer
<400> 78
   cacyggatgg ccaatccaa 19
<210> 79
   <211> 17
   <212> DNA
   <213> Artificial Sequence
<220>
   <223> primer
<400> 79
   aagagyctat tgagcta 17
<210> 80
   <211> 16
   <212> DNA
   <213> Artificial Sequence
<220>
   <223> primer
<400> 80
   ccaccttctt ccccat 16
<210> 81
   <211> 17
   <212> DNA
   <213> Artificial Sequence
<220>
   <223> primer
<400> 81
   ctcatkggct ggaagtt 17
<210> 82
   <211> 22
   <212> DNA
   <213> Artificial Sequence
<220>
   <223> primer
<400> 82
   cctcgaagca gtaagtgcgg tc 22
<210> 83
   <211> 20
   <212> DNA
   <213> Artificial Sequence
<220>
   <223> primer
<400> 83
   ggctcctggc atcactactg 20
<210> 84
   <211> 223
   <212> DNA
   <213> Enterovirus sp.
<400> 84
   aagagtctat tgagctagtt agtagtcctc cggcccctga atgcggctaa tcctaactgc 60
   ggagcacata ccctcgaccc agggggcagt gtgtcgtaac gggcaactct gcagcggaac 120
   cgactacttt gggtgtccgt gtttcctttt attcttatac tggctgctta tggtgacaat 180
   tgaaagattg ttaccatata gctattggat tggccatccg gtg 223
<210> 85
   <211> 326
   <212> DNA
   <213> Enterovirus 71
<400> 85
   gggtggtact ggaagttccc agatgtactg actgaaaccg gagtttttgg tccaaatgca     60
   caatttcact acttataccg ttcagggttc tgcatccacg ttcaatgtaa cgctagcaaa    120
   tttcaccaag gggcgctact cgttgcggta ttgcccgagt atgtcattgg aacagtggca    180
   ggcggcacag gcacagagaa cagtcaccct ccttataaac aaacccaacc cggcgctgat    240
   ggatttgaat tacaacatcc atatgttctt gatgctggaa ttccaatatc tcagttgaca    300
   gtgtgccctc accagtggat caattt                                         326
<210> 86
   <211> 326
   <212> DNA
   <213> Coxsackievirus A16
<400> 86
   ggatggtatt ggaaattccc tgatgtcttg acagaagtag gtgtttttgg tcaaaatgct     60
   cagttccact acctgtatcg atccgggttt tgcgtgcacg ttcagtgtaa tgcgagtaaa    120
   ttccaccagg gcgctttact ggtggctgtg ctgcccgagt atgtgctcgg cactatcgca    180
   ggagggaccg ggaatgagaa ttctcatcct ccctacgcca ctacacagcc tggtcaggtt    240
   ggtgcagttc tgacgcatcc ttacgtgtta gatgcaggga ttcctttgag ccaattgact    300
   gtgtgccccc accaatggat caactt                                         326
<210> 87
   <211> 556
   <212> DNA
   <213> Adenovirus sp.
<400> 87
   ccaccttctt ccccatggcg cacaacaccg cctccacgct tgaggccatg ctgcgcaacg 60
   acaccaatga ccaatccttc aacgactacc tctcggcggc caacatgctc tatcccatcc 120
   cggccaacgc caccaacgtc gccatctcca tcccctcgcg caactgggcg gcctttcgcg 180
   gctggtcctt cacgcgactc aagaccaaag agacgccctc gctgggctcc gggttcgacc 240
   cctacttcgt ctactcgggc tccatcccct acctcgacgg caccttctac ctcaaccaca 300
   ccttcaagaa ggtctccatc accttcgact cttccgtcag ctggcccgtc aacgaccggc 360
   tcctgacgcc caacgagttc gaaatcaagc gcaccgtcga cggcgaggga tacaacgtgg 420
   cccagtgcaa catgaccaag gactggttcc tggtccagat gctggcccac tacaacatcg 480
   gctaccaggg cttctacgtg cccgagggct acaaggaccg catgtactcc ttcttccgca 540
   acttccagcc catgag 556
<210> 88
   <211> 313
   <212> DNA
   <213> Homo sapiens
<400> 88
   gtttgagacc ttcaacaccc cagccatgta cgttgctatc caggctgtgc tatccctgta 60
   cgcctctggc cgtaccactg gcatcgtgat ggactccggt gacggggtca cccacactgt 120
   gcccatctac gaggggtatg ccctccccca tgccatcctg cgtctggacc tggctggccg 180
   ggacctgact gactacctca tgaagatcct caccgagcgc ggctacagct tcaccaccac 240
   ggccgagcgg gaaatcgtgc gtgacattaa ggagaagctg tgctacgtcg ccctggactt 300
   cgagcaagag atg 313
<210> 89
   <211> 420
   <212> DNA
   <213> Hepatitis E virus
<400> 89
   ggctcctggc atcactactg ctattgagca ggctgctcta gcagcggcca actctgccct 60
   tgcgaatgct gtggtagtta ggccttttct ctctcaccag cagattgaga tccttattaa 120
   cctaatgcaa cctcgccagc ttgttttccg ccccgaggtt ttctggaacc atcccatcca 180
   gcgtgttatc cataatgagc tggagcttta ctgtcgcgcc cgttccggcc gctgccttga 240
   aattggtgcc cacccccgct caataaatga taaccctaat gtggtccacc gctgcttcct 300
   ccgccctgcc gggcgtgatg ttcagcgttg gtatactgcc cctacccgcg ggccggctgc 360
   taattgccgg cgttccgcac tgcgcgggct ccccgctgct gaccgcactt actgcttcga 420
<210> 90
   <211> 246
   <212> DNA
   <213> Hepatitis A virus
<400> 90
   gttttgctcc tctttatcat gctatggatg ttactacaca agttggagat gattctggag    60
   gtttttcaac aacagtttct acagaacaga atgttccaga tccccaagtt ggtatgacaa   120
   ccatgaaaga tttgaaagga aaagctaaca gagggaaaat ggatgtttca ggagtacaag   180
   cacctgtggg agctatcaca acaattgagg atccagtttt agcaaagaaa gtacctgaga   240
   catttc                                                              246
<210> 91
   <211> 470
   <212> DNA
   <213> Norwalk virus sp.
<400> 91
   ataaaagttg gcatgaacac aatagaagat ggccccctca tctatgctga gcatgctaaa    60
   tataagaatc attttgatgc agattataca gcatgggact caacacaaaa tagacaaatt   120
   atgacagaat ccttctccat tatgtcgcgc cttacggcct caccagaatt ggccgaggtt   180
   gtggcccaag atttgctagc accatctgag atggatgtag gtgattatgt catcagggtc   240
   aaagaggggc tgccatctgg attcccatgt acttcccagg tgaacagcat aaatcactgg   300
   ataattactc tctgtgcact gtctgaggcc actggtttat cacctgatgt ggtgcaatcc   360
   atgtcatatt tctcatttta tggtgatgat gagattgtgt caactgacat agattttgac   420
   ccagcccgcc tcactcaaat tctcaaggaa tatggcctca aaccaacaag               470
<210> 92
   <211> 392
   <212> DNA
   <213> Rotavirus sp.
<400> 92
   ggctttaaaa gagagaattt ccgtctggtt agcggttagc tccttttaat gtatgggatc    60
   gaatatacaa cggcactaac ctttttgata tcattcattt tactgaacta tacacttaag   120
   tctttgacta aaatgatgga ctttgtcatt tatagatttc ttttcgttat agttgtcttg   180
   tcaccattgc taaaagcaca gaattatgga ataaacctgc caatcactgg ttcaatggat   240
   actgcgtacg ctaattccac acaagaagag gcactcctta cgtctactct atgcctgtac   300
   tatcctactg aagctgcaac agaaataaat gataattcat ggaaagatac gctttcacag   360
   ttgtttctga ctaagggatg gcctactgga tc                                 392
<210> 93
   <211> 23
   <212> DNA
   <213> Artificial Sequence
<220>
   <223> primer
<400> 93
   ggtctcaacc ccatccgtgt cgg 23
<210> 94
   <211> 25
   <212> DNA
   <213> Artificial Sequence
<220>
   <223> primer
<400> 94
   tgcggaaagc tgtatttcta cagtt 25
<210> 95
   <211> 20
   <212> DNA
   <213> Artificial Sequence
<220>
   <223> primer
<400> 95
   ccctcgacca agccaacctc 20
<210> 96
   <211> 21
   <212> DNA
   <213> Artificial Sequence
<220>
   <223> primer
<400> 96
   tgcgcgttgt tcttgttggt g 21
<210> 97
   <211> 20
   <212> DNA
   <213> Artificial Sequence
<220>
   <223> primer
<400> 97
   accgaacagc aaatgaaaga 20
<210> 98
   <211> 20
   <212> DNA
   <213> Artificial Sequence
<220>
   <223> primer
<400> 98
   aacgcctggc ttgtttttgt 20
<210> 99
   <211> 17
   <212> DNA
   <213> Artificial Sequence
<220>
   <223> primer
<400> 99
   aagagyctat tgagcta 17
<210> 100
   <211> 19
   <212> DNA
   <213> Artificial Sequence
<220>
   <223> primer
<400> 100
   cacyggatgg ccaatccaa 19
<210> 101
   <211> 30
   <212> DNA
   <213> Artificial Sequence
<220>
   <223> probe
<400> 101
   gaattaaacc acatgctcca ctgcttgtgc 30
<210> 102
   <211> 26
   <212> DNA
   <213> Artificial Sequence
<220>
   <223> probe
<400> 102
   aatcccgact cgttaaagca ggataa 26
<210> 103
   <211> 30
   <212> DNA
   <213> Artificial Sequence
<220>
   <223> probe
<400> 103
   aacaagtttc agaaagattt gatggcaaag 30
<210> 104
   <211> 30
   <212> DNA
   <213> Artificial Sequence
<220>
   <223> probe
<400> 104
   gagccggaaa tcgtgcgcga catcaaggag 30
<210> 105
   <211> 195
   <212> DNA
   <213> Chlamydia pneumoniae
<400> 105
   tgcggaaagc tgtatttcta cagttgtcaa atacatgtca agtccaggta aggtccttcg 60
   cgttgcatcg aattaaacca catgctccac tgcttgtgcg ggcccccgtc aattcttttg 120
   agtttcaccc ttgcgagtgt actcctcagg cggcatactt aacacgttag ctccgacacg 180
   gatggggttg agacc 195
<210> 106
   <211> 323
   <212> DNA
   <213> Mycoplasma pneumoniae
<400> 106
   ccctcgacca agccaacctc cagctctgaa cgggggcggg gtgaaggaat gataaggctt 60
   caagtggaca aagtgacgaa aaccacacca agttcacgag cgctacgggg atggaccagc 120
   agggacaatc aggtacctcc gcggggaatc ccgactcgtt aaagcaggat aatattagta 180
   agagtgggga tagtttaacc acgcaggacg gcaatgcgat cgatcaacaa gaggccacca 240
   actacaccaa cctccccccc aacctcaccc ccaccgctga ttgaccgaac gcgctgtcat 300
   tcaccaacaa gaacaacgcg cag 323
<210> 107
   <211> 144
   <212> DNA
   <213> Legionella pneumophila
<400> 107
   accgaacagc aaatgaaaga cgttcttaac aagtttcaga aagatttgat ggcaaagcgt 60
   actgctgaat tcaataagaa agcggatgaa aataaagtaa aaggggaagc ctttttaact 120
   gaaaacaaaa acaagccagg cgtt 144
<210> 108
   <211> 313
   <212> DNA
   <213> Homo sapiens
<400> 108
   gtttgagacc ttcaacaccc cagccatgta cgttgctatc caggctgtgc tatccctgta 60
   cgcctctggc cgtaccactg gcatcgtgat ggactccggt gacggggtca cccacactgt 120
   gcccatctac gaggggtatg ccctccccca tgccatcctg cgtctggacc tggctggccg 180
   ggacctgact gactacctca tgaagatcct caccgagcgc ggctacagct tcaccaccac 240
   ggccgagcgg gaaatcgtgc gtgacattaa ggagaagctg tgctacgtcg ccctggactt 300
   cgagcaagag atg 313